(19) Europäisches Patentamt
European Patent Office
Office européen des brevets

(11) Publication number: **0 330 992 A2**

# (12) EUROPEAN PATENT APPLICATION

(21) Application number: **89103121.3**

(22) Date of filing: **23.02.89**

(51) Int. Cl.[4]: **C07D 239/54 , C07D 473/34 , C07D 473/18 , C07D 473/40 , C07D 303/04 , C07D 473/16 , C07D 473/32 , C07D 473/06 , A61K 31/52**

(30) Priority: **29.02.88 JP 44553/88**
**08.07.88 JP 168784/88**

(43) Date of publication of application:
**06.09.89 Bulletin 89/36**

(84) Designated Contracting States:
**DE FR GB IT**

(71) Applicant: **Nippon Kayaku Kabushiki Kaisha**
**11-2, Fujimi-cho 1 chome Chiyoda-ku**
**Tokyo(JP)**

(72) Inventor: **Ichikawa, Yuh-ichiro**
**3-17-1-301, Shimo Kita-ku**
**Tokyo(JP)**
Inventor: **Yamazaki, Masanori**
**2716, Ooazakawarabuki**
**Ageo-shi Saitamaken(JP)**
Inventor: **Matsuo, Kaoru**
**2-4, Tomisato**
**Kashiwa-shi Chiba-ken(JP)**
Inventor: **Aoyama, Keiko**
**3-7-2-302, Shimo Kita-ku**
**Tokyo(JP)**
Inventor: **Matsumura, Fumiko**
**1-8-10, Tomioka**
**Urayasu-shi Chiba-ken(JP)**
Inventor: **Nishiyama, Yukihiro**
**2-8-25, Shiratori Togo-cho Aichi-gun**
**Aichi-ken(JP)**
Inventor: **Matsubara, Kenichi**
**3-18-1-804, Yamadahigashi**
**Suita-shi Oosaka-fu(JP)**
Inventor: **Nagahata, Takemitsu**
**2-6-25, Kamishinden**
**Toyonaka-shi Oasaka-fu(JP)**
Inventor: **Hoshino, Hiroo**
**1-14-5, Heiwa-cho**
**Maebashi-shi Gunma-ken(JP)**
Inventor: **Seki, Junichi**
**239, Iwahana-machi**
**Takasaki-shi Gunmaken(JP)**

(74) Representative: **Türk, Gille, Hrabal**
**Bruckner Strasse 20**
**D-4000 Düsseldorf 13(DE)**

## (54) Novel cyclobutane Derivatives, processes for their preparation and pharmaceutical compositions comprising them.

(57) The invention is directed to cyclobutane derivatives represented by general formula (I):

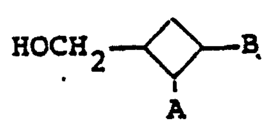

(I)

wherein A represents a hydrogen atom, a hydroxy group, an azido group or an amino group; and B represents a purine residue or a pyrimidine residue. The cyclobutane derivatives exhibit antiviral and antitumor activities and are thus expected as drugs.

EP 0 330 992 A2

# NOVEL CYCLOBUTANE DERIVATIVES; PROCESSES FOR THEIR PREPARATION AND PHARMACEUTICAL COMPOSITIONS COMPRISING THEM

## BACKGROUND OF THE INVENTION

### Field of the Invention

The present invention relates to cyclobutane derivatives which are expected as drugs, for example, antiviral agents, etc.

### Description of the Prior Art

Many substances associated with nucleic acid are known to have an antiviral activity and some of them have been provided for clinical use as useful drugs. As antiviral agents, there are known, for example, Vidarabine (M. Privat de Garilhe and J. de Rubber, C.R. Acad. Soc. D (Paris), 259, 2725 (1964)), Aciclovir (G.B. Elion et al., Proc. Natl. Acad. Sci. USA, 74, 5716 (1977)), Azidothymidine (H. Mitsuya et al., Proc. Natl. Acad. Sci. USA, 82, 7096 (1985)), etc.

With respect to the antiviral agents described above, however, many problems remain unsolved in that they merely have a narrow range of application, mode of administration is limited due to affecting solubility, oral absorption, metabolism, etc., difficulty arises in long term administration because of side effects such as bone marrow supression, etc. It is the situation that many viral diseases, for example, viral hepatitis, viral digestive diseases and respiratory diseases, etc. have no appropriate antiviral agent but rely on symptomatic therapy. Furthermore, malignant viral diseases such as acquired immunodeficiency syndrome (AIDS), adult T cell leukemia (ATL), etc., tend to increase so that development on excellent antiviral agents has been demanded.

## SUMMARY OF THE INVENTION

The present invention relates to cyclobutane derivatives represented by general formula (I):

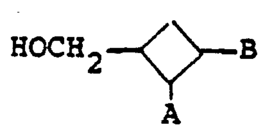

(I)

wherein A represents a hydrogen atom, a hydroxy group, an azido group or an amino group; and B represents a purine residue or a pyrimidine residue.

## DETAILED DESCRIPTION OF THE INVENTION

As the purine residue in general formula (I) described above, mention may be made of those obtained by removing the hydrogen atom at the 1-, 7- or 9-position of nucleic acid type purine bases such as:

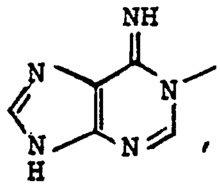 , 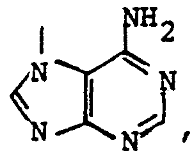 , 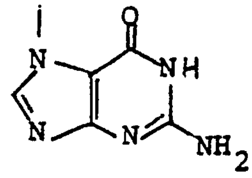 , 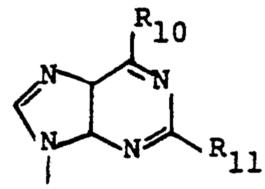

wherein $R_{10}$ represents a hydrogen atom, an amino group or a halogen atom and $R_{11}$ represents a hydrogen atom or an amino group, or,

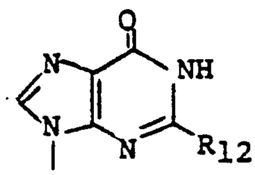

wherein $R_{12}$ represents a hydrogen atom or an amino group.

As the pyrimidine residue, mention may be made of residues obtained by removing the hydrogen atom at the 1-position of nucleic acid type pyrimidine bases such as :

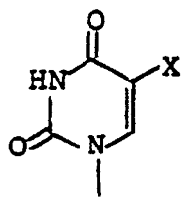 or 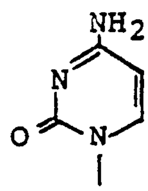

wherein X represents a hydrogen atom, a halogen atom or a lower alkyl group.

Preferred examples in the general formula described above are residues wherein A is a hydroxy group and B is:

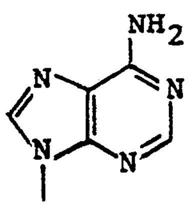 or 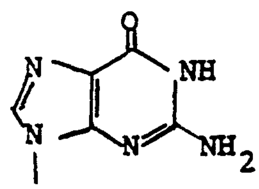

As the compounds of the present invention represented by general formula (I), the following compounds are illustrated, wherein in terms relative steric configuration, when cyclobutane is considered to be planar surface, a substituent positioned below the plane is shown by α and a substituent positioned above the plane is shown by β.

| No. | Compound |
| --- | --- |
| 1. | (±)-1-[(1β,2α,3β)-2-Hydroxy-3-hydroxymethylcyclobutan-1-yl]-2,4(1H,3H)-pyrimidinedione and optically active compounds thereof |
| 2. | (±)-5-Fluoro-1-[(1β,2α,3β)-2-hydroxy-3-hydroxymethylcyclobutan-1-yl]-2,4(1H,3H)-pyrimidinedione and optically active compounds thereof |
| 3. | (±)-1-[(1β,2α,3β)-2-Hydroxy-3-hydroxymethylcyclobutan-1-yl]-5-methyl-2,4(1H,3H)-pyrimidinedione and optically active compounds thereof |
| 4. | (±)-4-Amino-1-[(1β,2α,3β)-2-hydroxy-3-hydroxymethylcyclo-clobutan-1-yl]-2(1H)-pyrimidinone and optically active compounds thereof |
| 5. | (±)-1-[(1β,2α,3β)-2-Hydroxy-3-hydroxymethylcyclobutan-1-yl]adenine and optically active compounds thereof |
| 6. | (±)-7-[(1β,2α,3β)-2-Hydroxy-3-hydroxymethylcyclobutan-1-yl]adenine and optically active compounds thereof |
| 7. | (±)-9-[(1β,2α,3β)-2-Hydroxy-3-hydroxymethylcyclobutan-1-yl]adenine and optically active compounds thereof |
| 8. | (±)-7-[(1β,2α,3β)-2-Hydroxy-3-hydroxymethylcyclobutan-1-yl]guanine and optically active compounds thereof |

9. (±)-9-[(1β,2α,3β)-2-Hydroxy-3-hydroxymethylcyclobutan-1-yl]guanine and optically active compounds thereof

10. (±)-5-Fluoro-1-[(1β,2β,3β)-2-hydroxy-3-hydroxymethylcyclobutan-1-yl]-2,4(1H,3H)-pyrimidinedione and optically active compounds thereof

11. (±)-1-[(1β,2β,3β)-2-Hydroxy-3-hydroxymethylcyclobutan-1-yl]-5-methyl-2,4(1H,3H)-pyrimidinedione and optically active compounds thereof

12. (±)-1-[(1α,2β,3β)-2-Hydroxy-3-hydroxymethylcyclobutan-1-yl]-2,4(1H,3H)-pyrimidinedione and optically active compounds thereof

13. (±)-5-Fluoro-1-[(1α,2β,3β)-2-hydroxy-3-hydroxymethylcyclobutan-1-yl]-2,4(1H,3H)-pyrimidinedione and optically active compounds thereof

14. (±)-1-[(1β,2α,3β)-2-Azido-3-hydroxymethylcyclobutan-1-yl]-5-fluoro-2,4(1H,3H)-pyrimidinedione and optically active compounds thereof

15. (±)-1-[(1β,2α,3β)-2-Azido-3-hydroxymethylcyclobutan-1-yl]-5-methyl-2,4(1H,3H)-pyrimidinedione and optically active compounds thereof

16. (±)-1-[(1β,2α,3β)-2-Amino-3-hydroxymethylcyclobutan-1-yl]-5-fluoro-2,4(1H,3H)-pyrimidinedione and optically active compounds thereof

17. (±)-1-[(1β,2α,3β)-2-Amino-3-hydroxymethylcyclobutan-1-yl]-5-methyl-2,4(1H,3H)-pyrimidinedione and optically

active compounds thereof

18. (±)-1-[(1β,2β,3β)-2-Hydroxy-3-hydroxymethylcyclobutan-1-yl]-2,4(1H,3H)-pyrimidinedione and optically active compounds thereof

19. (±)-4-Amino-1-[(1β,2β,3β)-2-hydroxy-3-hydroxymethylcyclobutan-1-yl]-2(1H)-pyrimidinone and optically active compounds thereof

20. (±)-9-[(1β,2β,3β)-2-Hydroxy-3-hydroxymethylcyclobutan-1-yl]adenine and optically active compounds thereof

21. (±)-9-[(1β,2β,3β)-2-Hydroxy-3-hydroxymethylcyclobutan-1-yl]guanine and optically active compounds thereof

22. (±)-1-[(1β,2α,3β)-2-Azido-3-hydroxymethylcyclobutan-1-yl]-2,4(1H,3H)-pyrimidinedione and optically active compounds thereof

23. (±)-4-Amino-1-[(1β,2α,3β)-2-azido-3-hydroxymethylcyclobutan-1-yl]-2(1H)-pyrimidinone and optically active compounds thereof

24. (±)-9-[(1β,2α,3β)-2-Azido-3-hydroxymethylcyclobutan-1-yl]adenine and optically active compounds thereof

25. (±)-9-[(1β,2α,3β)-2-Azido-3-hydroxymethylcyclobutan-1-yl]guanine and optically active compounds thereof

26. (±)-1-[(1β,2α,3β)-2-Amino-3-hydroxymethylcyclobutan-1-yl]-2,4(1H,3H)-pyrimidinedione and optically active compounds thereof

27. (±)-4-Amino-[(1β,2α,3β)-2-amino-3-hydroxymethylcyclobutan-1-yl]-5-methyl-2(1H)-pyrimidinone and optically active compounds thereof

28. (±)-9-[(1β,2α,3β)-2-Amino-3-hydroxymethylcyclobutan-1-yl]adenine and optically active compounds thereof

29. (±)-9-1-[(1β,2α,3β)-2-Amino-3-hydroxymethylcyclobutan-1-yl]guanine and optically active compounds thereof

2-1. 9-[(1β,3β)-3-Hydroxymethylcyclobutan-1-yl]adenine

2-2. 9-[(1β,3β)-3-Hydroxymethylcyclobutan-1-yl]guanine

2-3. 9-[(1α,3β)-3-Hydroxymethylcyclobutan-1-yl]adenine

2-4. 9-[(1α,3β)-3-Hydroxymethylcyclobutan-1-yl]guanine

3-19. 1-[(1β,3β)-3-Hydroxymethylcyclobutan-1-yl]-2,4(1H,3H)-pyrimidinedione

3-20. 5-Fluoro-1-[(1β,3β)-3-hydroxymethylcyclobutan-1-yl]-2,4(1H,3H)-pyrimidinedione

3-21. 1-[(1β,3β)-3-Hydroxymethylcyclobutan-1-yl]-5-methyl-2,4(1H,3H)-pyrimidinedione

3-22. 4-Amino-1-[(1β,3β)-3-hydroxymethylcyclobutan-1-yl]-2H(1H)-pyrimidinone

4-1. (±)-2-Amino-9-[(1β,2α,3β)-2-hydroxy-3-hydroxymethylcyclobutan-1-yl]purine and optically active compounds thereof

4-2. (±)-9-[(1β,2α,3β)-2-Hydroxy-3-hydroxymethylcyclobutan-1-yl]hypoxanthine and optically active compounds thereof

4-3. (±)-2-Amino-6-chloro-9-[(1β,2α,3β)-2-hydroxy-3-hydroxymethylcyclobutan-1-yl]purine and optically active compounds thereof

4-4. (±)-2,6-Diamino-9-[(1β,2α,3β)-2-hydroxy-3-hydroxymethylcyclobutan-1-yl]purine and optically active compounds thereof

Among the compounds of the present invention represented by general formula (I), compounds represented by general formula (IA):

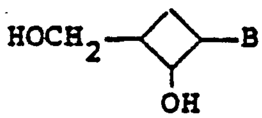

(IA)

wherein B has the same significance as defined for B in general formula (I), can be produced as follows.

That is, the compounds (IA) can be produced by reacting cyclobutane derivatives represented by general formula (II):

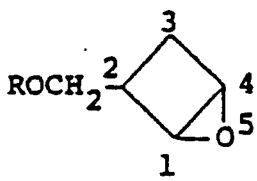

(II)

wherein R represents a hydrogen atom or a protective group, with compounds represented by general formulae (III) - (X):

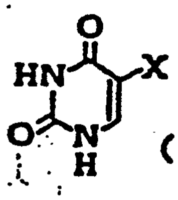

(III)

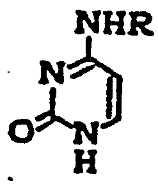

(IV)

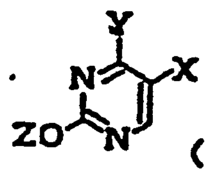

(V)

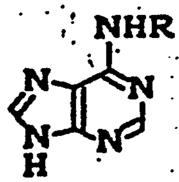

(VI)

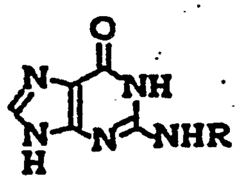

(VII)

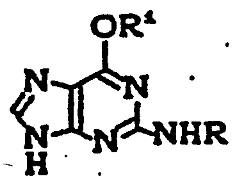

(VIII)

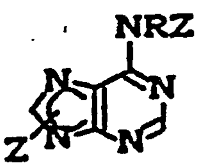

(IX)

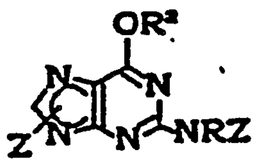

(X)

wherein X has the same significance as defined in general formula (I); Y represents O-silyl group or NR-silyl group; Z represents silyl group; $R^1$ represents R or an alkyl group; and $R^2$ represents $R^1$ or Z, to give compounds represented by general formula (XIA):

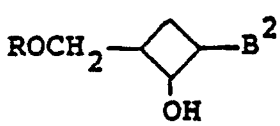

(XIA)

wherein $B^2$ represents

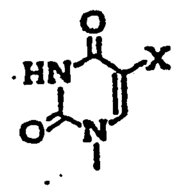

(wherein X represents a hydrogen atom, a halogen atom or a lower alkyl group) or

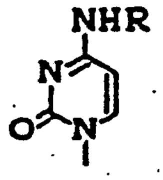
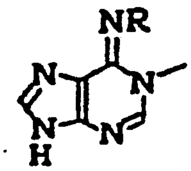
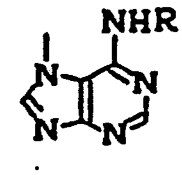
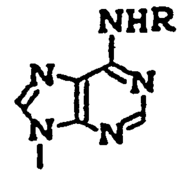
;

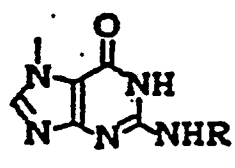
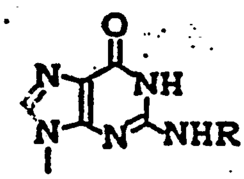
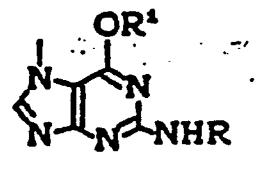
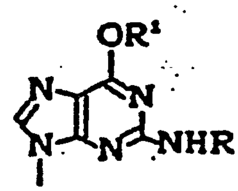

(wherein R represents a hydrogen atom or a protective group and R[1] represents R or an alkyl group); then removing the remaining protective group or, subjecting the compounds represented by general formula (XIA) either to the Mitsunobu method (O. Mitsunobu, Synthesis, 1 (1981)) or to oxidation-reduction, etc. to invert the hydroxy atom at the 2′-position and removing the protective group of the resulting compounds represented by general formula (XIA) using appropriate chemicals.

As the protective group (R) in the general formula (II), there is no particular restriction so long as it is generally usable as a protective group; examples of such a protective group are an acyl group such as acetyl group, benzoyl group, etc.; a carbamoyl group such as dimethylcarbamoyl group, diphenylcarbamoyl group, etc.; a silyl ether type protective group such as t-butyldimethylsilyl group, t-butyldiphenylsilyl group, etc.; or an ether type protective group such as methoxymethyl group, benzyl group, etc.

Examples of the compound represented by general formula (II) include the following compounds.

a. (±)-(1α,2β,4α)-2-Hydroxymethyl-5-oxabicyclo[2.1.0]pentane and optically active compounds thereof

b. (±)-(1α,2β,4α)-2-Acetoxymethyl-5-oxabicyclo[2.1.0]pentane and optically active compounds thereof

c. (±)-(1α,2β,4α)-2-Benzoyloxymethyl-5-oxabicyclo[2.1.0]pentane and optically active compounds thereof

d. (±)-(1α,2β,4α)-2-t-Butyldimethylsilyloxymethyl-5-oxabicyclo[2.1.0]pentane and optically active compounds thereof

e. (±)-(1α,2β,4α)-2-t-Butyldiphenylsilyloxymethyl-5-oxabicyclo[2.1.0]pentane and optically active compounds thereof

f. (±)-(1α,2β,4α)-2-Benzyloxymethyl-5-oxabicyclo[2.1.0]pentane and optically active compounds thereof

g. (±)-(1α,2β,4α)-2-Methoxymethoxymethyl-5-oxabicyclo[2.1.0]pentane and optically active compounds thereof

h. (±)-(1β,2β,4β)-2-Hydroxymethyl-5-oxabicyclo[2.1.0]pentane and optically active compounds thereof

i. (±)-(1β,2β,4β)-2-Acetoxymethyl-5-oxabicyclo[2.1.0]pentane and optically active compounds thereof

j. (±)-(1β,2β,4β)-2-Benzoyloxymethyl-5-oxabicyclo[2.1.0]pentane and optically active compounds thereof

k. (±)-(1β,2β,4β)-2-t-Butyldimethylsilyloxymethyl-5-oxabicyclo[2.1.0]pentane and optically active compounds thereof

l. (±)-(1β,2β,4β)-2-t-Butyldiphenylsilyloxymethyl-5-oxabicyclo[2.1.0]pentane and optically active compounds thereof

m. (±)-(1β,2β,4β)-2-Benzyloxymethyl-5-oxabicyclo[2.1.0]pentane and optically active compounds thereof

n. (±)-(1β,2β,4β)-2-Methoxymethoxymethyl-5-oxabicyclo[2.1.0]pentane and optically active compounds thereof

As the silyl group in general formulae (III) to (X), mention may be made of trimethylsilyl group, etc.; the protective group is the same as in the protective group in the general formula (II). As the alkyl group, mention may be made of butyl group, benzyl group, 2-methoxyethoxy group, etc.

Further compounds represented by general formulae (V), (IX) and (X) can be prepared from the corresponding compounds represented by general formulae (III), (V) and (VI) to (VIII) by known methods (T. Nishimura and I. Iwai, Chem. Pharm. Bull., 12, 352 (1964), R. Zou and M.J. Robins, Can. J. Chem., 65, 1436 (1987)).

A proportion of the compound of general formula (II) and the compounds represented by general formulae (III) to (X) is desirably approximately 0.5 to 10 molar equivalents, preferably approximately 1 to 4 molar equivalents, of the latter, based on 1 molar equivalent of the former. The reaction between these compounds is carried out in the presence of an acidic catalyst or a basic catalyst. The reaction is performed in an organic solvent such as methylene chloride, chloroform, dichloroethane, benzene, toluene, chlorobenzene, acetonitrile, etc. at -30 °C to a reflux temperature of the solvent, preferably at about 0 °C to about 30 °C, using as the acidic ctalyst a Lewis acid such as boron trifluoride etherate ($BF_3.OEt_2$), tin tetrachloride, titanium tetrachloride, zinc chloride, magnesium bromide, etc. or an acid such as trimethylsilyl trifluoromethanesulfonate, ethansulfonic acid, trifluoromethanesulfonic acid, etc. It is preferred that the acidic catalyst be used in an amount of approximately 0.1 to 5-fold molar equivalents, preferably 1 to 2-fold molar equivalents, based on the compound of general formula (II). The reaction is also performed in a solvent such as dimethylformamide, dimethylsulfoxide, 1,3-dimethyl-2-imidazolinone, hexamethylphosphoric triamide, etc., at room temperature to a reflux temperature of the solvent, preferably at about 80 to about 200 °C, using as the basic ctalyst potassium carbonate, lithium hydride, sodium hydride, etc. It is preferred that the basic catalyst be used in an amount of approximately 0.01 to 5-fold molar equivalents, preferably 0.1 to 1.2-fold molar equivalents, based on the compound of general formula (II).

The compounds represented by general formula (II) can be produced by epoxydation of compounds represented by general formula (XII):

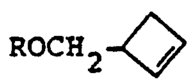

(XII)

wherein R has the same significance as defined in general formula (II).

The epoxydation can be effected either by reacting the compound of general formula (XII) with a peracid such as performic acid, peracetic acid, m-chloroperbenzoic acid, monoperphthalic acid, etc. in a solvent such as water, formic acid, acetic acid, an ether, benzene, toluene, methylene chloride, chloroform, etc., or a solvent mixture thereof, at 0 °C to a reflux temperature of the solvent, or by reacting oxazilidines shown by formula (XIII) to (XVI):

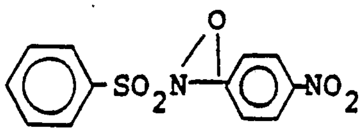

(XIII)

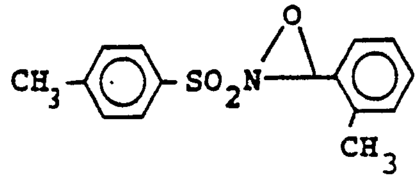

(XIV)

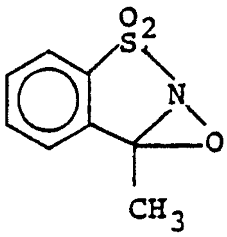

(XV)

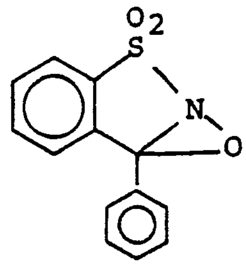

(XVI)

in an organic solvent such as benzene, chloroform, acetonitrile, etc. at 0°C to a reflux temperature of the solvent.

Among the compounds of the present invention represented by general formula (I), compounds represented by general formula (IB):

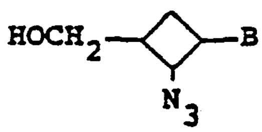

(IB)

wherein B has the same significance as defined for B in general formula (I), can be produced as follows. That is, the compounds (IB) can be produced by activating the hydroxy group at the 2′-position of the compounds represented by general formula (XIA) through sulfonylation such as methanesulfonylation, trifluoromethanesulfonylation, p-toluenesulfonylation, etc. or halogenation such as bromination, iodination, etc., then reacting the activated hydroxy group with azide ions such as ions of sodium azide, potassium azide, etc., and then removing the protective group of compounds represented by general formula (XIB):

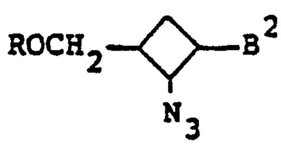

(XIB)

wherein $B^2$ and R have the same significances as defined in general formula (XIA) using appropriate chemicals.

Among the compounds of the present invention represented by general formula (I), compounds represented by general formula (IC):

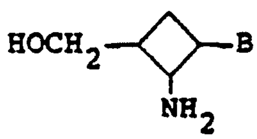

(IC)

wherein B has the same significance as defined for B in general formula (I), can be produced as follows. That is, the compounds (IC) can be produced by reducing the azide group represented by general formula

(XIB) with appropriate reducing agents such as catalytic reduction agents, etc. and then removing the protective group of the resulting compounds represented by general formula (XIC):

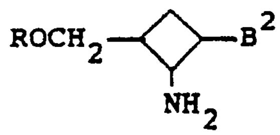

(XIC)

wherein $B^2$ and R have the same significances as defined in general formula (XIA) using appropriate chemicals.

Among the compounds of the present invention represented by general formula (I), compounds represented by general formula (ID):

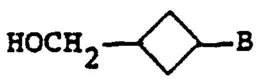

(ID)

wherein B has the same significance as defined for B in general formula (I), can be produced as follows. That is, the compounds (ID) can be produced by reacting compounds represented by general formula (XVII):

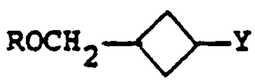

(XVII)

wherein R has the same significance as defined in general formula (XI) and Y represents a leaving group, with compounds represented by general formulae (III) through (X), and then removing the protective group of the resulting compounds represented by general formula (XID):

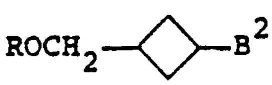

(XID)

wherein $B^2$ and R have the same significances as defined in general formula (XIA) using appropriate chemicals.

Examples of the leaving group (Y) in the general formula (XVII) include a sulfonyloxy group such as methanesulfonyloxy group, p-toluenesulfonyloxy group, trifluoromethanesulfonyloxy group, etc.; a halogen such as chlorine, bromine, iodine, etc.

A proportion of the compound of general formula (XVII) and the compounds represented by general formulae (III) to (X) is desirably approximately 0.5 to 10 molar equivalents, preferably approximately 1 to 4 molar equivalents, of the latter, based on 1 molar equivalent of the former. The reaction between these compounds is carried out in the presence or absence of a basic catalyst. The reaction is performed in a solvent such as dimethylformamide, dimethylsulfoxide, 1,3-dimethyl-2-imidazolinone, hexamethylphosphoric triamide, etc., at room temperature to a reflux temperature of the solvent, preferably at about 40 to about 170 °C, using as the basic ctalyst potassium carbonate, lithium hydride, sodium hydride, etc. It is preferred that the basic catalyst be used in an amount of approximately 0.1 to 5-fold molar equivalents, preferably 1.0 to 1.2-fold molar equivalents, based on the compound of general formula (XVII).

The compound represented by general formula (XVII) can be prepared from the compound represented by general formula (II). That is, the compound of formula (XVII) can be prepared by reducing the compound represented by general formula (II) (R = H) with a reducing agent such as lithium aluminum hydride, sodium bis(2-metoxyethoxy)aluminum hydride, etc., introducing a protective group into the primary hydroxy group of the resulting compound represented by general formula (XVIII):

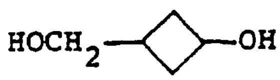 (XVIII)

and leading the secondary hydroxy group to a leaving group.

The protective group (R) in the general formulae (XIA) to (XID) is not particularly limited so long as it is generally usable as the protective group. Examples of such a protective group include an acyl group such as acetyl group, benzoyl group, etc.; a carbamoyl group such as dimethylcarbamoyl group, diphenylcarbamoyl group, etc.; a silyl ether type protective group such as t-butyldimethylsilyl group, t-butyldiphenylsilyl group, etc.; or an ether type protective group such as methoxymethyl group, benzyl group, etc. Further in case that two or more R groups are present in the same molecule, each R may be the same or different. The protective group in the compounds represented by general formulae (XIA) to (XID) can be removed by appropriate chemicals and methods for removing protective groups depending upon kind of the protective group in combination. Examples of such chemicals and methods are an alkali such as sodium hydroxide, sodium methylate, ammonia, etc., a fluorine agent such as tetrabutyl ammonium fluoride, etc., an acid such as hydrochloric acid, etc., and hydrogenolysis, etc.

Compounds in the general formula (I) described above wherein A is a hydrogen atom and B is a pyrimidine residue are also produced by the following steps using the compound represented by general formula (XVII):

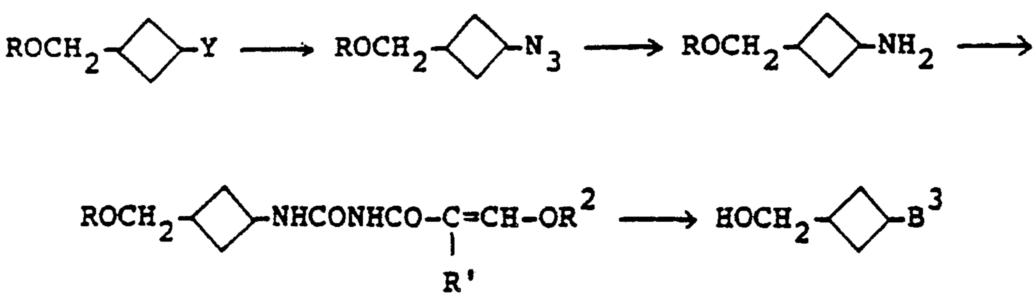

wherein R and Y have the same significances as defined above, R′ is a hydrogen atom or methyl group, $R^2$ is a lowr alkyl group having 1 to 4 carbon atoms and $B^3$ represents a pyrimidine residue.

Further compounds represented by general formula (I) described above wherein B represents:

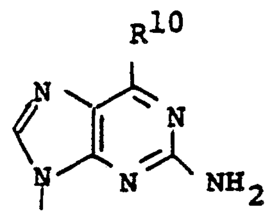

wherein $R_{10}$ has the same significances as defined above, can be produced, for example, by the following steps:

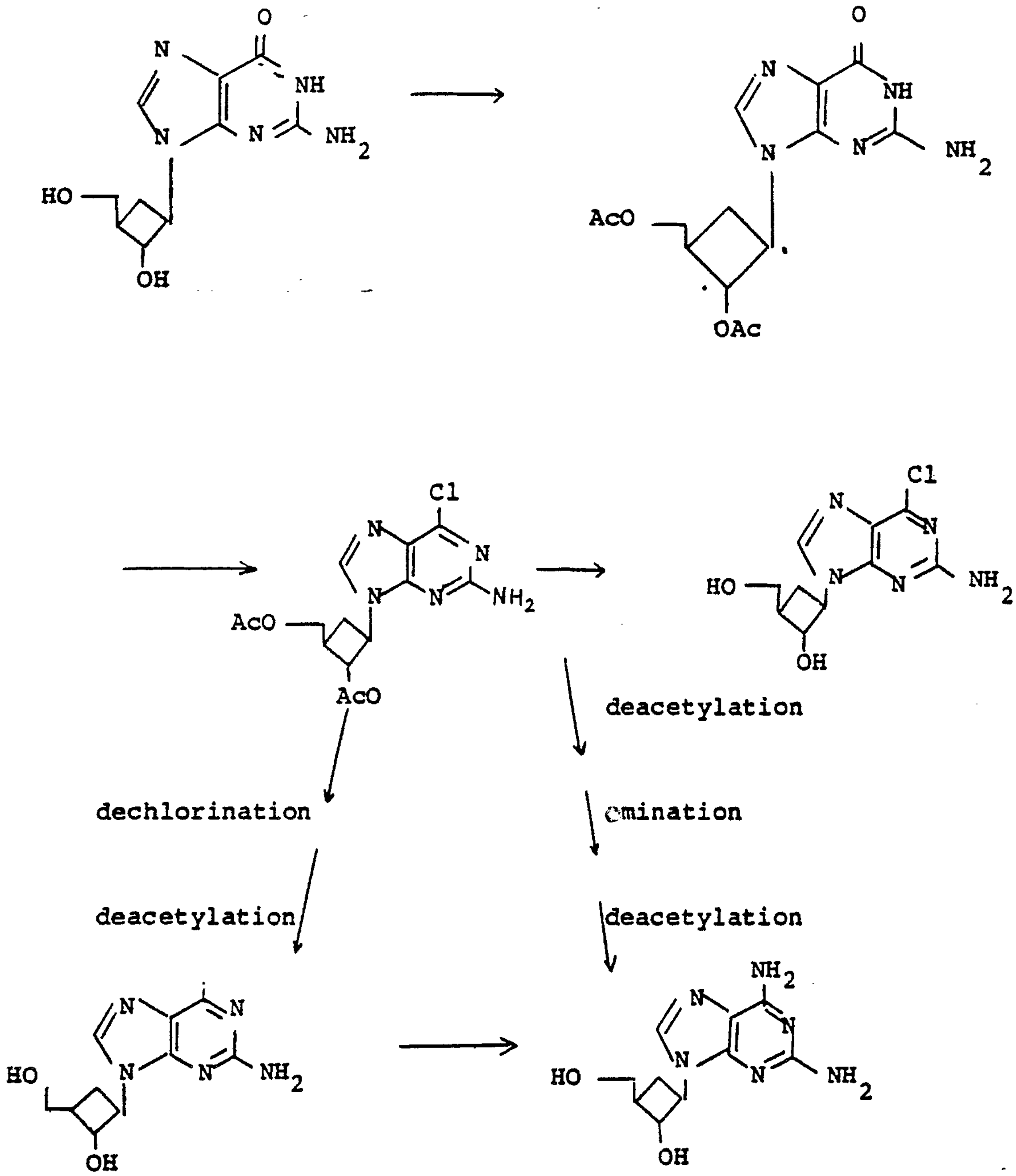


Further compounds represented by general formula (I) described above wherein B represents:

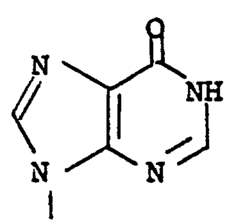

can also be prepaerd by the following process:

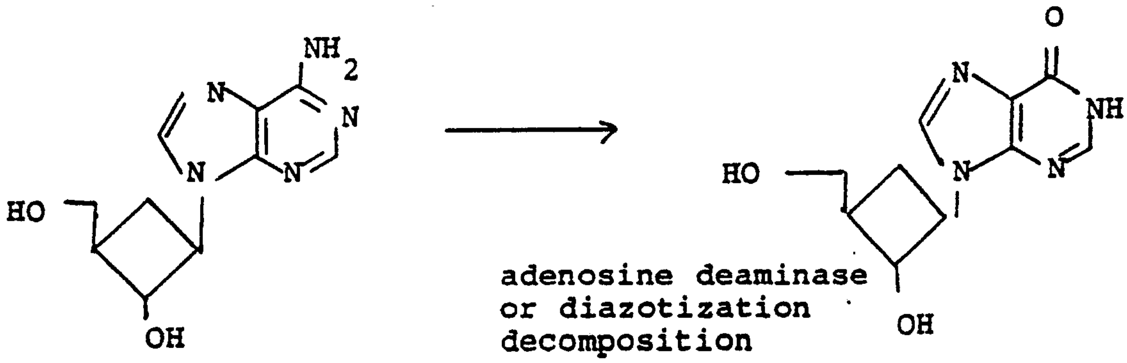


The compounds represented by general formula (I) contain asymmetric carbon therein. The present invention also includes these racemi and optically active compounds. The optically active compounds can be obtained from the racemi compounds in a conventional manner, for example, a method using an optically active column chromatography, a method which comprises reacting an optically active resolution auxiliary agent and separating and purifying the resulting diastereomer, etc. Further by using the compounds of general formula (II) which are optically active, the compounds of general formula (I) which are optically active can also be obtained.

The compounds represented by general formula (I) may be converted into the acid addition salt thereof, if necessary and desired. Examples of the acid addition salt include inorganic acid salts such as hydrochlorides, hydrobromides, sulfates, phosphates, etc.; and organic acid salts such as formates, acetates, citrates, tartrates, maleates, fumarates, lactates, methanesulfonates, etc.

It is noted from the following test examples that the compounds of the present invention exhibit antiviral and antitumor activities.

Test Example 1

Antiviral activity against herpes simplex virus type 1 (HSV-I) and herpes simplex virus type 2 (HSV-II) which were DNA viruses was examined by the following methods.

(Method 1)

A 6 well multiplate bearing a single layr of Vero cells (derived from African green monkey kidney cell) was infected with 100-150 PFU (plaque forming unit) of virus. After (infection) at 37°C for an hour, agar medium (Eagle's MEM medium supplemented with 1.5% agar) containing a sample of each concentration was put on the single layer followed by culturing at 37°C for 48 hours in a 5% (v/v) carbon dioxide incubator. Thereafter, formation of plaque was measured to determine 50% inhibitory concentration ($IC_{50}$).

Test Example 2

An activity against human cytomegalovirus (HCMV) which was DNA virus was examined by the following method.

(Method 2)

Anticytomegaloviral activity was determined in terms of 50% inhibitory concentration ($IC_{50}$) by infecting 35 mm dish having a single layer of human fetal fibroblast with 100 PFU (plaque forming unit) of cytomegalovirus (AO169 strain), adsorbing for an hour, laying thereon a medium containing the compound of each concentration supplemented with 0.5% agarose and 2% fetal calf serum, culturing at 37°C for 10 days in a 5% (v/v) carbon dioxide gas incubator and measuring formation of plaque.

Test Example 3

Activity against hepatitis B virus (HBV) which was DNA virus was examined by the following method.

(Method 3)

Incubated liver cell line HB611 (Proc. Natl. Acad. Sci. USA, 84 (1987), 444) capable of producing and releasing activated hepatitis B virus was cultured in modified Eagle's medium (GIBCO) at 37° C in 5% $CO_2$ in the presence 10% fetal calf serum, 200 µg/ml of G418 and 100 U/ml each of penicillin and streptomycin. The culture was inoculated on a 6 well plate in 5 x $10^4$ cells/well (35 mm). When 50% confluent state was achieved 1 or 2 days after, a definite quantity of this compound was added and the mixture was cultured. While exchanging with a medium containing the compound in the same concentration every 3 other days, incubation was continued for 15 days. Thereafter the medium was removed. The cells were treated with 0.5 ml of lysis buffer (10 mM Tris HCl, pH 7.8/5 mM $Na_2$EDTA, 1% SDS/0.1 mg/ml Pronase K) at 37° C for an hour. The resulting DNA was treated with RNase and purified by treatment with phenol and chloroform and then precipitation with ethanol.

Then, 5 µg of DNA was treated with Hind III and DNA pattern was analyzed by the Southern method using as a probe $^{32}$P-labeled hepatitis B virus DNA.

Test Example 4

Activity against AIDS virus (HIV) which was RNA virus was examined by the following method.

(Method 4)

In an anti-HIV (human immunodeficiency virus) active 24 well tray were charged about 50,000/ml of MT-4 cells. Further 100 µg of a solution containing a definite amount of the compound of this invention was added thereto. After culturing at 37° C for 2 hours in a 5% (v/v) carbon dioxide gas incubator, $10^3$-$10^4$ infection units of HIV were added thereto. After culturing for 4 days, a part of the culture solution was smeared on a slide glass and fixed with acetone. Then, occurrence of viral antigen was observed by fluorescent antibody method.

As primary antibody in the fluorescent antibody method, serum from the patient with AIDS was used and FITC-labeled anti-human IgG was used as secondary antibody.

Test Example 5

Anticancer activity against human uterine cervical cancer HeLa $S_3$, mouse leukemia L1210 and P388 cells was examined by the following method.

(Method 5)

HeLa $S_3$ cells were prepared in a concentration of 7.5 x $10^3$ cells/ml and inoculated on a 96 well flat bottom plate by 0.2 ml/well each. After culturing at 37° C for 24 hours in a 5% $CO_2$ incubator, 10 µl of the compound was added thereto followed by culturing for 72 hours. After culturing, the culture medium of each well was withdrawn and fixed with methanol. Then, 0.1 ml/well each of 0.05% Methylene Blue/10 mM-Tris-HCl (pH 8.5) solution was added to stain at room temperature for 30 minutes. After withdrawing the dyeing solution from each well using an aspirator, the well was washed 3 times with pure water. After adding 3% HCl in a ratio of 0.2 ml/well, the well was sealed and allowed to stand at room temperature for about 24 hours and the dye was then extracted from the cells. Absorbance of each well at 660 nm was measured with Dynatec microplate reader. The growth inhibition rate treated wells to the control wells were calculated according to the equation below and 50% growth inhibitory concentration ($IC_{50}$, µg/ml) was determined by the Litchfield method.

Growth inhibition rate (%) = $(1 - A_1/A_0) \times 100$

wherein
$A_1$ = Absorbance of the treated group
$A_0$ = Absorbance of the control group

(Method 6)

P388 and L1210 cells were inoculated on a 24 well plate and the compound was added thereto followed by incubation at 37° C for 48 hours in 5% $CO_2$. The number of cells after the incubation was counted with a Caulter counter. The growth inhibition rate of the treated group to the control group was calculated according to the equation below and 50% growth inhibitory concentration ($IC_{50}$, µg/ml) was determined by the Litchfield method.
Growth inhibition rate (%) = $[1 - (N_T-N_0)/(N_c-N_0)] \times 100$
wherein
$N_T$ = cell count of the treated group
$N_0$ = cell count at the onset of incubation
$N_c$ = cell count of the control group
The results of the tests described above are shown in Table 1.

Table 1

| | $IC_{50}$(µg/ml) | | | | | | | |
|---|---|---|---|---|---|---|---|---|
| Compound No. | HSV-1 | HSV-2 | HIV | HCMV | HBV | HeLaS3 | P338 | L1210 |
| 7 | 4.0 | | | 0.45 | | 6.97 | 1.64 | 1.84 |
| 9 | 0.30 | 0.31 | | 0.95 | | 10.9 | 2.57 | 2.56 |
| 14 | | | | 20 | 32.0 | | | |
| 17 | | | | | 35.0 | | | |
| 2-1 | | | 0.3 | | 2.0 | 100 | 34.1 | 63.1 |
| 2-2 | 1.6 | 3.6 | 10 | 2.6 | | 30.0 | 11.5 | 23.1 |
| 4-2 | | | | 2.8 | | | | |
| 4-3 | 8.5 | | | 3.3 | | 66 | 12 | 8.0 |
| 4-4 | 2.2 | | 3 | 0.69 | | 21 | 3.4 | 2.4 |

The compounds of the present invention represented by general formula (I) have an antiviral activity over a wide range and are thus expected to be effective for many viral diseases such as infections with lip or genital herpes, herpes zoster, herpes simplex virus type 1, type 2 (HSV-I, II) upon immunosuppression, varicella zoster virus (VZV), cytomegalovirus (CMV) or Epstein-Barr virus (EBV); viral hepatitis, virus-induced respiratory diseases, AIDS, ATL, etc. Furthermore, the compounds of the present invention are also expected as cancerocidal agents.

In case that the compounds of the present invention thus obtained are used as antiviral agents, they can be administered orally, intravenously or subcutaneously. Dosage may vary depending upon condition and age of the patient to be administered and mode of administration but is generally in a range of 0.1 to 500 mg/kg/day. The compounds of the present invention are mixed with appropriate carriers for pharmaceutical preparations and administered in the controlled preparation form. As the preparation form, tablets, granules, granulates, powders, capsules, injections, cream, suppositories, etc. are used.

Next, production of the compounds of the present invention is described more specifically with reference to the examples below.

Example 1

Preparation of (±)-1-[(1β,2α,3β)-2-hydroxy-3-hydroxymethylcyclobutan-1-yl]-2,4(1H,3H)-pyrimidinedione (Compound 1):

Step 1

Preparation of (±)-1-[(1β,2α,3β)-3-t-butyldiphenylsilyloxymethyl-2-hydroxycyclobutan-1-yl]-2,4(1H,3H)-pyrimidinedione

(±)-1-(1α,2β,4α)-2-t-Butyldiphenylsilyloxymethyl-5-oxabicyclo[2.1.0]pentane (Compound e) (339 mg, 1.0 mmol) and 2,4-bistrimethylsilyloxypyrimidine (1.03 g, 4.0 mmol) were dissolved in anhydrous methylene chloride (10 ml) and an anhydrous methylene chloride solution (1 ml) of boron trifluoride etherate (170 mg, 1.2 mmol) was added to the solution at room temperature in an argon atmosphere. The mixture was stirred for a day as it was. To the reaction mixture was added 0.2 M phosphate buffer (pH 7.0). After stirring for a while, extraction was performed with methylene chloride. After the extract was dried over anhydrous sodium sulfate, the solvent was distilled off under reduced pressure. The residue was purified by silica gel column chromatography (methylene chloride : methanol = 50 : 1 (v/v)) to give (±)-1-[(1β,2α,3β)-3-t-butyldiphenylsilyloxymethyl-2-hydroxycyclobutan-1-yl]-2,4(1H,3H)-pyrimidinedione (222 mg, 49%).
NMR (400 MHz.FT, DMSO-$d_6$) δ:
1.01 (9H, s), 1.49 (1H, apparent q, J=9.9Hz), 2.05 (1H, m), 2.12 (1H, apparent q, J=8.8Hz), 3.77 (2H, d, J=4.7Hz), 4.08 (1H, apparent q, J=7.3Hz, changed to apparent t(J=7.3Hz) after addition of $D_2O$), 4.41 (1H, apparent q, J=8.6Hz), 5.595 (1H, d, J=7.0Hz, exchangeable with $D_2O$), 5.601 (1H, d, J=8.1Hz), 7.38-7.52 (6H, m), 7.60-7.66 (4H, m), 7.66 (1H, d, J=8.1Hz), 11.27 (1H, s, exchangeable with $D_2O$).

Step 2

Preparation of (±)-1-[(1β,2α,3β)-2-hydroxy-3-hydroxymethylcyclobutan-1-yl]-2,4(1H,3H)-pyrimidinedione (Compound 1):

(±)-1-[(1β,2α,3β)-3-t-Butyldiphenylsilyloxymethyl-2-hydroxycyclobutan-1-yl]-2,4(1H,3H)-pyrimidinedione (90 mg, 0.20 mmol) was dissolved in methanol (1 ml) and 4N-hydrochloric acid/dioxane (0.1 ml, 0.4 mmol) was added to the solution. The mixture was stirred at room temperature overnight. After the reaction liquid was concentrated, water was added thereto and ether-soluble matters were removed from the mixture followed by purification by means of Sephadex LH-20 column chromatography (methanol). The purified product was dissolved in water and then freeze dried to give (±)-1-[(1β,2α,3β)-2-hydroxy-3-hydroxymethylcyclobutan-1-yl]-2,4(1H,3H)-pyrimidinedione (Compound 1) (42 mg, 99%).
NMR (400 MHz.FT, $CD_3OD + D_2O$) δ:
1.60 (1H, apparent q, J=9.8Hz), 2.14 (1H, m), 2.30 (1H, apparent q, J=9.3Hz), 3.71 (2H, m), 4.16 (1H, apparent t, J=7.0Hz), 4.47 (1H, apparent q, J=8.4Hz), 5.80 (1H, d, J=8.1Hz), 7.67 (1H, d, J=8.1Hz).

Example 2

Preparation of (±)-5-fluoro-1-[(1β,2α,3β)-2-hydroxy-3-hydroxymethylcyclobutan-1-yl]-2,4(1H,3H)-pyrimidinedione (Compound 2):

Step 1

Preparation of (±)-1-[(1β,2α,3β)-3-t-butyldiphenylsilyloxymethyl-2-hydroxycyclobutan-1-yl]-5-fluoro-2,4-(1H,3H)-pyrimidinedione

(±)-(1α,2β,4α)-2-t-Butyldiphenylsilyloxymethyl-5-oxabicyclo[2.1.0]pentane (Compound e) (135 mg, 0.40 mmol) and 2,4-bistrimethylsilyloxy-5-fluoropyrimidine (329 mg, 1.20 mmol) were dissolved in anhydrous methylene chloride (4 ml) and an anhydrous methylene chloride solution (1 ml) of boron trifluoride etherate (68 mg, 48 mmol) was added to the solution at room temperature in an argon atmosphere. The mixture was stirred for a day as it was. To the reaction mixture was added 0.2 M phosphate buffer (pH 7.0). After stirring

for a while, extraction was performed with methylene chloride. After the extract was dried over anhydrous sodium sulfate, the solvent was distilled off under reduced pressure. The residue was purified by silica gel column chromatography (methylene chloride : methanol = 50 : 1 (v/v)) to give (±)-1-[(1β,2α,3β)-3-t-butyldiphenylsilyloxymethyl-2-hydroxycyclobutan-1-yl]-5-fluoro-2,4(1H,3H)-pyrimidinedione (140 mg, 75%).
NMR (400 MHz.FT, $CDCl_3$) δ:
1.07 (9H, s), 1.71 (1H, m), 2.13-2.27 (2H, m), 3.51 (1H, brs), 3.74 (1H, dd, J=3.0Hz, J=11.0Hz), 3.84 (1H, dd, J=3.3Hz, J=11.0Hz), 4.20 (1H, m), 7.20 (1H, d, J=5.8Hz), 7.33-7.50 (6H, m), 7.60-7.70 (4H, m), 9.25 (1H, brs).

Step 2

Preparation of (±)-5-fluoro-1-[(1β,2α,3β)-2-hydroxy-3-hydroxymethylcyclobutan-1-yl]-2,4(1H,3H)-pyrimidinedione (Compound 2):

(±)-1-[(1β,2α,3β)-3-t-Butyldiphenylsilyloxymethyl-2-hydroxycyclobutan-1-yl]-5-fluoro-2,4(1H,3H)-pyrimidinedione (140 mg, 0.30 mmol) was dissolved in methanol (2 ml) and 4N-hydrochloric acid/dioxane (0.15 ml, 0.6 mmol) was added to the solution. The mixture was stirred at room temperature overnight. After the reaction liquid was concentrated, ether-soluble matters were removed from the mixture followed by purification by means of Sephadex LH-20 column chromatography (methanol). The purified product was dissolved in water and then freeze dried to give (±)-5-fluoro-1-[(1β,2α,3β)-2-hydroxy-3-hydroxymethylcyclobutan-1-yl]-2,4(1H,3H)-pyrimidinedione (Compound 2) (67 mg, 97%).
NMR (400 MHz.FT, $CD_3OD$) δ:
1.57 (1H, apparent q, J=10.3Hz), 2.05 (1H, m), 2.24 (1H, apparent q, J=9.0Hz), 3.60-3.75 (2H, m), 4.10 (1H, apparent t, J=7.7Hz), 4.46 (1H, apparent q, J=8.7Hz), 7.86 (1H, d, J=6.6Hz).

Example 3

Preparation of (±)-1-[(1β,2α,3β)-2-Hydroxy-3-hydroxymethylcyclobutan-1-yl]-5-methyl-2,4(1H,3H)-pyrimidinedione (Compound 3):

Step 1

Preparation of (±)-1-[(1β,2α,3β)-3-t-butyldiphenylsilyloxymethyl-2-hydroxycyclobutan-1-yl]-5-methyl-2,4-(1H,3H)-pyrimidinedione

(±)-1-(1α,2β,4α)-2-t-Butyldiphenylsilyloxymethyl-5-oxabicyclo[2.1.0]pentane (Compound e) (169 mg, 0.50 mmol) and 2,4-bistrimethylsilyloxy-5-methylpyrimidine (541 mg, 2.00 mmol) were dissolved in anhydrous methylene chloride (5 ml) and an anhydrous methylene chloride solution (1 ml) of boron trifluoride etherate (85 mg, 0.60 mmol) was added to the solution at room temperature in an argon atmosphere. The mixture was stirred for a day as it was. To the reaction mixture was added 0.2 M phosphate buffer (pH 7.0). After stirring for a while, extraction was performed with methylene chloride. After the extract was dried over anhydrous sodium sulfate, the solvent was distilled off under reduced pressure. The residue was purified by silica gel column chromatography (methylene chloride : methanol = 50 : 1 (v/v)) to give (±)-1-[(1β,2α,3β)-3-t-butyldiphenylsilyloxymethyl-2-hydroxycyclobutan-1-yl]-5-methyl-2,4(1H,3H)-pyrimidinedione (153 mg, 66%).
NMR (400 MHz.FT, $CDCl_3$) δ:
1.07 (9H, s), 1.75 (1H, m), 1.93 (3H, s), 2.15-2.30 (2H, m), 3.66 (1H, brs), 3.76 (1H, apparent d, J=10.6Hz), 3.84 (1H, apparent d, J=10.6Hz), 4.15 (2H, m), 6.95 (1H, s), 7.33-7.50 (6H, m), 7.60-7.70 (4H, m), 8.94 (1H, br).

Step 2

Preparation of (±)-1-[(1β,2α,3β)-2-hydroxy-3-hydroxymethylcyclobutan-1-yl]-5-methyl-2,4(1H,3H)-pyrimidinedione (Compound 3):

(±)-1-[(1β,2α,3β)-3-t-Butyldiphenylsilyloxymethyl-2-hydroxycyclobutan-1-yl]-2,4(1H,3H)-pyrimidinedione (142 mg, 0.31 mmol) was dissolved in methanol (1 ml) and 4N-hydrochloric acid/dioxane (0.15 ml, 0.6 mmol) was added to the solution. The mixture was stirred at room temperature overnight. After the reaction liquid was concentrated, ether-soluble matters were removed from the mixture followed by purification by means of Sephadex LH-20 column chromatography (methanol). The purified product was dissolved in water and then freeze dried to give (±)-1-[(1β,2α,3β)-2-hydroxy-3-hydroxymethylcyclobutan-1-yl]-5-methyl-2,4-(1H,3H)-pyrimidinedione (Compound 3) (67 mg, 97%).
NMR (400 MHz.FT, DMSO-$d_6$) δ:
1.40 (1H, apparent q, J=9.9Hz), 1.80 (3H, s), 1.90 (1H, m), 2.03 (1H, apparent q, J=8.8Hz), 3.43-3.60 (2H, m), 3.97 (1H, apparent q, J=7.5Hz, changed to apparent t(J=7.7Hz) after addition of $D_2O$), 4.41 (1H, apparent q, J=8.6Hz), 5.57 (1H, t, J=5.3Hz, exchangeable with $D_2O$), 5.47 (1H, d, J=6.6Hz, exchangeable with $D_2O$), 7.55 (1H, s), 11.24 (1H, brs, exchangeable with $D_2O$).

Example 4

Preparation of (±)-4-amino-1-[(1β,2α,3β)-2-hydroxy-3-hydroxymethylcyclobutan-1-yl]-2(1H)-pyrimidinone (Compound 4)

Step 1

Preparation of (±)-4-amino-1-[(1β,2α,3β)-3-t-butyldiphenylsilyloxymethyl-2-hydroxycyclobutan-1-yl]-2(1H)-pyrimidinone

(±)-(1α,2β,4α)-2-t-Butyldiphenylsilyloxymethyl-5-oxabicyclo[2.1.0]pentane (Compound e) (178 mg, 0.53 mmol) and bistrimethylsilyl-N-acetylcytosine (476 mg, 1.60 mmol) were dissolved in anhydrous methylene chloride (5 ml) and an anhydrous methylene chloride solution (1 ml) of boron trifluoride etherate (90 mg, 0.63 mmol) was added to the solution at room temperature in an argon atmosphere. The mixture was stirred for a day as it was. To the reaction mixture was added 0.2 M phosphate buffer (pH 7.0). After stirring for a while, extraction was performed with methylene chloride. After the extract was dried over anhydrous sodium sulfate, the solvent was distilled off under reduced pressure. The residue was purified by silica gel column chromatography (methylene chloride : methanol = 50 : 1 and then 30 : 1 (v/v)) to give (±)-4-acetylamino-1-[(1β,2α,3β)-3-t-butyldiphenylsilyloxymethyl-2-hydroxycyclobutan-1-yl]-2(1H)-pyrimidinone (83 mg, 32%).
NMR (400 MHz.FT, $CDCl_3$) δ:
1.03 (9H, s), 1.83 (1H, m), 2.20-2.40 (2H, m), 2.25 (3H, s), 3.75 (1H, dd, J=2.7Hz, J=10.8Hz), 3.83 (1H, dd, J=3.5Hz, J=10.8Hz), 4.11 (1H, apparent t, J=6.8Hz), 4.20 (1H, apparent q, J=7.5Hz), 4.54 (1H, s), 7.32-7.54 (8H, m), 7.57-7.70 (4H, m), 8.86 (1H, brs).

Step 2

Preparation of (±)-4-amino-1-[(1β,2α,3β)-2-hydroxy-3-hydroxymethylcyclobutan-1-yl]-2(1H)-pyrimidinone (Compound 4)

(±)-4-Acetylamino-1-[(1β,2α,3β)-3-t-butyldiphenylsilyloxymethyl-2-hydroxycyclobutan-1-yl]-2(1H)-pyrimidinone (76 mg, 0.16 mmol) was dissolved in methanol (2 ml) and 1M-sodium methoxide/methanol (0.2 ml, 0.2 mmol) was added to the solution. The mixture was stirred at room temperature for 30 minutes. Amberlyst 15 (42 mg, 0.21 mmol) was added to the reaction liquid. After stirring for 10 minutes, the resin was filtered off followed by concentration. The residue was dissolved in tetrahydrofuran (2 ml) and 1M-tetrabutyl ammonium fluoride/tetrahydrofuran (0.25 ml, 0.25 mmol) was added to the solution under ice cooling. The mixture was stirred at room temperature for 7 hours. The reaction liquid was diluted with

methanol and Amberlyst 15 (52 mg, 0.25 mmol) was added to the dilution. After stirring the mixture, the resin was filtered off and the solvent was removed by distillation. After ether soluble matters were removed, the residue was purified by Sephadex LH-20 column chromatography (methanol) to give (±)-4-amino-1-[-(1β,2α,3β)-2-hydroxy-3-hydroxymethylcyclobutan-1-yl]-2(1H)-pyrimidinone (Compound 4) (36 mg, quantitative).

NMR (400 MHz.FT, DMSO-$d_6$) δ:
1.57 (1H, m), 1.89 (1H, m), 2.04 (1H, apparent q, J=9.0Hz), 3.40-3.56 (2H, m), 3.90 (1H, m), 4.39 (1H, apparent q, J=8.8Hz), 4.56 (1H, brs, exchangeable with $D_2O$), 5.39 (1H, brs, exchangeable with $D_2O$), 5.70 (1H, d, J=7.3Hz), 7.01 (2H, brd, exchangeable with $D_2O$), 7.56 (1H, d, J=7.3Hz).

Example 5

Preparation of (±)-1-[(1β,2α,3β)-2-hydroxy-3-hydroxymethylcyclobutan-1-yl]adenine (Compound 5):

Step 1

Preparation of (±)-6-N-benzoyl-1-[(1β,2α,3β)-2-hydroxy-3-hydroxymethylcyclobutan-1-yl]adenine (Compound A) and (±)-6-N-benzoyl-7-[(1β,2α,3β)-2-hydroxy-3-hydroxymethylcyclobutan-1-yl]adenine (Compound B)

Bistrimethylsilyl-N-benzoyladenine synthesized from N-benzoyladenine (4.24 g, 17.7 mmol) according to the method described in T. Nishimura and I. Iwai, Chem. Pharm. Bull., 12, 352 (1964) and (±)-(1α,2β,4α)-2-t-butyldiphenylsilyloxymethyl-5-oxabicyclo[2.1.0]pentane (Compound e) (1.50 g, 4.43 mmol) were dissolved in anhydrous acetonitrile (20 ml) and trimethylsilyl trifluoromethanesulfonate (1.18 g, 5.3 mmol) was added to the solution at 0°C in an argon atmosphere. The mixture was stirred for 3 hours at the same temperature. To the reaction mixture was added 0.2 M phosphate buffer (pH 7.0) (100 ml). The mixture was extracted with ethyl acetate. After the extract was dried over anhydrous sodium sulfate, the solvent was distilled off under reduced pressure. The residue was dissolved in anhydrous tetrahydrofuran and 1M-tetrabutylammonium fluoride/tetrahydrofuran (35 ml, 35 mmol) was added to the solution at 0°C. The mixture was stirred for 5 hours at the same temperature. After the reaction mixture was concentrated under reduced pressure, the concentrate was separated and purified by silica gel column chromatography (methylene chloride : methanol = 15 : 1 and then 10 : 1 (v/v)). Thereafter crude crystals were recrystallized from methanol to give (±)-6-N-benzoyl-1-[(1β,2α,3β)-2-hydroxy-3-hydroxymethylcyclobutan-1-yl]adenine (Compound A) (0.52 g, 35%) and (±)-6-N-benzoyl-7-[(1β,2α,3β)-2-hydroxy-3-hydroxymethylcyclobutan-1-yl]-adenine (Compound B) (0.41 g, 27%).

(Compound A)

NMR (400 MHz.FT, DMSO-$d_6$) δ:
1.63 (1H, apparent q, J=10.1Hz), 2.37 (1H, m), 2.46 (1H, overlapped with solvent signals), 3.50-3.63 (2H, m), 4.32-4.53 (2H, m, changed to t(1H, J=7.7Hz) after addition of $D_2O$), 5.03 (1H, apparent q, J=8.2Hz), 5.60 (1H, brs, exchangeable with $D_2O$), 7.48 (2H, t, J=7.7Hz), 7.54 (1H, t, J=7.7Hz), 8.16 (1H, s), 8.17 (2H, d, J=7.7Hz), 8.54 (1H, s), 12.36 (1H, br, exchangeable with $D_2O$).

(Compound B)

NMR (400 MHz.FT, $CD_3OD$) δ:
2.19 (1H, m), 2.25 (1H, m), 2.50 (1H, m), 3.65-3.79 (2H, m), 4.09 (1H, apparent t, J=7.3Hz), 4.67 (1H, apparent q, J=8.4Hz), 7.47 (2H, t, J=7.5Hz), 7.60 (1H, t, J=7.5Hz), 8/01 (2H, d, J=7.5Hz), 8.22 (1H, s), 8.27 (1H, s).

Step 2

Preparation of (±)-1-[(1β,2α,3β)-2-hydroxy-3-hydroxymethylcyclobutan-1-yl]adenine (Compound 5)

Compound (A) (142 mg, 0.42 mmol) prepared in Step 1 was suspended in absolute methanol (20 ml) and sodium methoxide (45 mg, 0.83 mmol) was added to the suspension. The mixture was stirred at room temperature overnight and then at 50 °C for 15 hours. After the solvent was distilled off under reduced pressure, the residue was dissolved by adding water and ether thereto. Ether soluble matters were removed from the solution. The aqueous phase was purified by Sephadex HP-20 ($H_2O$-70% MeOH/$H_2O$, linear gradient) to give (±)-1-[(1β,2α,3β)-2-hydroxy-3-hydroxymethylcyclobutan-1-yl]adenine (Compound 5) (92 mg, 93%).
NMR (400 MHz.FT, $CD_3OD$) δ:
2.02 (1H, apparent q, J=10.1Hz), 2.27 (1H, m), 2.60 (1H, apparent q, J=8.9Hz), 3.65-3.77 (2H, m), 4.16 (1H, apparent t, J=7.7Hz), 4.43 (1H, apparent q, J=8.4Hz), 7.95 (1H, s), 8.06 (1H, s), 8.55 (1H, s).
UV $\lambda_{max}(H_2O)$ nm:
pH 1, 258; pH 7, 267; pH 13, 272.

Example 6

Preparation of (±)-7-[(1β,2α,3β)-2-hydroxy-3-hydroxymethylcyclobutan-1-yl]adenine (Compound 6):

Compound (B) (126 mg, 0.37 mmol) prepared in Example 5, Step 1 was suspended in absolute methanol (10 ml) and sodium methoxide (40 mg, 0.74 mmol) was added to the suspension. The mixture was stirred at room temperature overnight and then at 50 °C for 3 hours. After the solvent was distilled off under reduced pressure, the residue was dissolved by adding water and ether thereto. Ether soluble matters were removed from the solution. The aqueous phase was purified by Sephadex HP-20 (water-70% MeOH aqueous solution, linear gradient) to give (±)-7-[(1β,2α,3β)-2-hydroxy-3-hydroxymethylcyclobutan-1-yl]adenine (Compound 6) (80 mg, 92%).
NMR (400 MHz.FT, $CD_3OD$) δ:
2.17 (1H, apparent q, J=9.9Hz), 2.25 (1H, m), 2.51 (1H, apparent q, J=8.8Hz), 3.67-3.76 (2H, m), 4.09 (1H, apparent t, J=7.3Hz), 4.68 (1H, apparent q, J=8.3Hz), 8.21 (1H, s), 8.27 (1H, s).
UV $\lambda_{max}(H_2O)$ nm:
pH 1, 273; pH 7, 270; pH 13, 270.

Example 7

Preparation of (±)-9-[(1β,2α,3β)-2-hydroxy-3-hydroxymethylcyclobutan-1-yl]adenine (Compound 7)

Step 1

Preparation of (±)-9-[(1β,2α,3β)-3-t-butyldiphenylsilyloxymethyl-2-hydroxycyclobutan-1-yl]adenine

Adenine (973 mg, 7.2 mmol) was suspended in dimethylformamide (50 ml) in an argon atmosphere and 50% sodium hydride (58 mg, 1.2 mmol) was added to the suspension. The mixture was stirred at room temperature for an hour. (±)-(1α,2β,4α)-2-t-Butyldiphenylsilyloxymethyl-5-oxabicyclo[2.1.0]pentane (Compound e) (2.03 g, 6.0 mmol) was added to the suspension. The mixture was stirred for 18 hours at 140 °C. After the solvent was distilled off under reduced pressure, the residue was purified by silica gel column chromatography (methylene chloride : methanol = 20 : 1 (v/v)) to give (±)-9-[(1β,2α,3β)-3-t-butyldiphenylsilyloxymethyl-2-hydroxycyclobutan-1-yl]adenine (1.73 g, 61%).
NMR (400 MHz.FT, $CD_3OD$) δ:
1.09 (9H, s), 2.16 (1H, apparent q, J=9.6Hz), 2.25 (1H, m), 2.39 (1H, apparent q, J=8.8Hz), 3.84 (1H, dd, J=3.7Hz, J=10.6Hz), 3.91 (1H, dd, J=4.2Hz, J=10.8Hz), 4.55-4.80 (2H, m), 7.34-7.50 (6H, m), 7.62-7.78 (4H, m), 8.10 (1H, s), 8.17 (1H, s).

Step 2

Preparation of (±)-9-[(1β,2α,3β)-2-hydroxy-3-hydroxymethylcyclobutan-1-yl]adenine (Compound 7)

(±)-1-[(1β,2α,3β)-3-t-Butyldiphenylsilyloxymethyl-2-hydroxycyclobutan-1-yl]adenine (155 mg, 0.33 mmol) was dissolved in methanol (1 ml) and 4N-hydrochloric acid/dioxane (0.15 ml, 0.6 mmol) was added to the solution. The mixture was stirred at room temperature overnight. After the solvent was distilled off under reduced pressure, water was added thereto. The mixture was neutralized with 0.1 N sodium hydroxide aqueous solution and ether-soluble matters were removed and purification was performed using Sephadex LH-20 (50% methanol aqueous solution) to give (±)-9-[(1β,2α,3β)-2-hydroxycyclobutan-1-yl]-adenine (Compound 7) (80 mg, quantitative).
NMR (400 MHz.FT, $CD_3OD$) δ:
1.97 (1H, apparent q, J=10.1Hz), 2.23 (1H, m), 2.45 (1H, apparent q, J=9.3Hz), 3.70-3.82 (2H, m), 4.47 (1H, apparent t, J=7.9Hz), 4.63 (1H, apparent q, J=8.6Hz), 8.20 (1H, s), 8.24 (1H, s).
UV $\lambda_{max}(H_2O)$ nm:
pH 1, 258; pH 7, 261; pH 13, 261.
HRMS (FAB) Calcd for $[C_{10}H_{13}N_5O_2+H]^+$; 236.1147. Found; 236.1138.

Example 8

Preparation of (±)-7-[(1β,2α,3β)-2-hydroxy-3-hydroxymethylcyclobutan-1-yl]guanine (Compound 8):

Step 1

Preparation of (±)-7-[(1β,2α,3β)-3-t-butyldiphenylsilyloxymethyl-2-hydroxycyclobutan-1-yl]guanine

Tristrimethylsilylguanine synthesized from guanine (405 mg, 2.7 mmol) according to the method described in T. Nishimura and I. Iwai, Chem. Pharm. Bull., 12, 352 (1964) and (±)-(1α,2β,4α)-2-t-butyldiphenylsilyloxymethyl-5-oxabicyclo[2.1.0]pentane (Compound e) (227 mg, 0.67 mmol) were dissolved in anhydrous acetonitrile (5 ml) and trimethylsilyl trifluoromethanesulfonate (155 µl, 0.80 mmol) was added to the solution at 0° C in an argon atmosphere. The mixture was stirred for 4 days at room temperature. To the reaction mixture were added 0.2 M phosphate buffer (pH 7.0) and methylene chloride. After insoluble matters were removed by filtration, the mixture was extracted with methylene chloride. After the extract was dried over anhydrous sodium sulfate, the solvent was distilled off under reduced pressure. The residue was dissolved in methanol (100 ml) and anhydrous potassium carbonate (93 mg, 0.67 mmol) was added to the solution at 0° C. The mixture was stirred for 1.5 hours at the same temperature. After insoluble matters were filtered off, then the reaction mixture was concentrated under reduced pressure, the solvent was distilled off under reduced pressure. The residue was separated and purified by silica gel column chromatography (methylene chloride : methanol = 10 : 1 and then 5 : 1 (v/v)) to give (±)-7-[(1β,2α,3β)-3-t-butyldiphenylsilyloxymethyl-2-hydroxycyclobutan-1-yl]guanine (84 mg, 26%).
NMR (400 MHz.FT, DMSO-$d_6$) δ:
1.01 (9H, s), 1.80 (1H, apparent q, J=10.0Hz), 2.12 (1H, m), 2.30 (1H, apparent q, J=9.0Hz), 3.75-3.93 (2H, m), 4.29 (1H, m), 4.61 (1H, apparent t, J=8.5Hz), 5.73 (1H, d, J=6.6Hz), 6.17 (2H, s), 7.35-7.52 (6H, m), 7.55-7.76 (4H, m), 8.00 (1H, s), 10.84 (1H, s).

Step 2

Preparation of (±)-7-[(1β,2α,3β)-2-hydroxy-3-hydroxymethylcyclobutan-1-yl]guanine (Compound 8)

(±)-7-[(1β,2α,3β)-3-t-Butyldiphenylsilyloxymethyl-2-hydroxycyclobutan-1-yl]guanine (127 mg, 0.26 mmol) was dissolved in tetrahydrofuran (8 ml) and 1 M-tetrabutyl ammonium fluoride/tetrahydrofuran (0.4

ml, 0.4 mmol) was added to the solution. The mixture was stirred at room temperature overnight. Water and ether were added to the reaction liquid. After ether soluble matters were removed, recrystallization from water was repeated to give (±)-7-[(1β,2α,3β)-2-hydroxy-3-hydroxymethylcyclobutan-1-yl]guanine (40 mg, 61%).

NMR (400 MHz.FT, DMSO-$d_6$) δ:
1.73 (1H, apparent q, J=10.0Hz), 1.97 (1H, m), 2.24 (1H, apparent q, J=9.0Hz), 3.45-3.65 (2H, m), 4.20 (1H, apparent q, J=7.1Hz, changed to apparent t(J=7.5Hz) after addition of $D_2O$), 4.50-4.62 (2H, m, changed to 4.59 (1H, apparent q, J=8.5Hz) after addition of $D_2O$), 5.65 (1H, d, J=6.2Hz, exchangeable with $D_2O$), 6.17 (2H, s, exchangeable with $D_2O$), 8.03 (1H, s), 10.83 (1H, s, exchangeable with$D_2O$).

UV $\lambda_{max}(H_2O)$ nm:
pH 1, 249, 274; pH 7, 250(sh), 284; pH 13, 242(sh), 280.

Example 9

Preparation of (±)-9-[(1β,2α,3β)-2-hydroxy-3-hydroxymethylcyclobutan-1-yl]guanine (Compound 9):

Step 1

Preparation of (±)-2-amino-9-[(1β,2α,3β)-3-t-butyldiphenylsilyloxymethyl-2-hydroxycyclobutan-1-yl]-6-(2-methoxy)ethoxypurine

2-Amino-6-(2-methoxy)ethoxypurine (2.04 g, 9.75 mmol) was dissolved in dimethylformamide (200 ml) in an argon atmosphere and lithium hydride (23 mg, 2.9 mmol) was added to the solution. The mixture was stirred at room temperature for an hour. (±)-(1α,2β,4α)-2-t-Butyldiphenylsilyloxymethyl-5-oxabicyclo[2.1.0]-pentane (Compound e) (3.30 g, 9.75 mmol) was added to the solution. The mixture was stirred for a day at 150°C. After the solvent was distilled off under reduced pressure, the residue was purified by silica gel column chromatography (methylene chloride : methanol = 50 : 1 (v/v)) to give (±)-2-amino-9-[(1β,2α,3β)-3-t-butyldiphenylsilyloxymethyl-2-hydroxycyclobutan-1-yl]-6-(2-methoxy)ethoxypurine (1.35 g, 25%).

NMR (400 MHz.FT, $CDCl_3$) δ:
1.04 (9H, s), 1.95 (1H, apparent q, J=9.3Hz), 2.27-2.44 (2H, m), 3.43 (3H, s), 3.67-3.88 (4H, m), 4.17-4.34 (2H, m), 4.47 (1H, brs), 4.65 (2H, t, J=5.0Hz), 4.89 (2H, brs), 7.28-7.47 (6H, m), 7.51 (1H, s), 7.56-7.69 (4H, m).

Step 2

Preparation of (±)-9-[(1β,2α,3β)-2-hydroxy-3-hydroxymethylcyclobutan-1-yl]guanine (Compound 9)

To (±)-2-amino-9-[(1β,2α,3β)-3-t-butyldiphenylsilyloxymethyl-2-hydroxycyclobutan-1-yl]-6-(2-methoxy)-ethoxypurine (1.35 g, 25 mmol) was added 2N-hydrochloric acid (50 ml). The mixture was heated to reflux for an hour. Water was added to the reaction liquid. After ether soluble matters were removed, water was distilled off. Water was added to the residue. After neutralizing with 2N-sodium hydroxide, recrystallization from water was repeated to give (±)-9-[(1β,2α,3β)-2-hydroxy-3-hydroxymethylcyclobutan-1-yl]guanine (Compound 9) (399 mg, 64%).

NMR (400 MHz.FT, DMSO-$d_6$) δ:
1.64 (1H, apparent q, J=10.0Hz), 2.00 (1H, m), 2.19 (1H, apparent q, J=9.2Hz), 3.45-3.63 (2H, m), 4.18 (1H, m, changed to apparent t(J=7.7Hz) after addition of $D_2O$), 4.30 (1H, apparent q, J=8.7Hz), 4.57 (1H, brs, exchangeable with $D_2O$), 5.55 (1H, d, J=5.1HZ exchangeable with $D_2O$), 6.43 (2H, s, exchangeable with $D_2O$), 7.25 (1H, br, exchangeable with $D_2O$), 7.80 (1H, s).

UV $\lambda_{max}(H_2O)$ nm:
pH 1, 255, 279(sh); pH 7, 253, 273(sh); pH 13, 258(sh), 267.
HRMS (FAB) Calcd for $[C_{10}H_{13}N_5O_3+H]^+$; 252.1096. Found ; 252.1178.

Example 10

Preparation of (±)-5-fluoro-1-[(1β,2β,3β)-2-hydroxy-3-hydroxymethylcyclobutan-1-yl]-2,4(1H,3H)-pyrimidinedione (Compound 10)

Step 1

Preparation of (±)-1-[(1β,2β,3β)-3-t-butyldiphenylsilyloxymethyl-2-hydroxycyclobutan-1-yl]-5-fluoro-2,4-(1H,3H)pyrimidinedione

In an argon atmosphere, dimethylsulfoxide (583 µl, 8.2 mmol) was gradually added to a solution of oxalyl chloride (350 µl, 4.1 mmol) in tetrahydrofuran (9 ml) at -78° C. Then, the temperature was raised to -35° C and stirring was continued for 5 minutes at the same temperature. The reaction liquid was again cooled to -78° C. After a solution of (±)-1-[(1β,2α,3β)-3-t-butyldiphenylsilyloxymethyl-2-hydroxycyclobutan-1-yl]-5-fluoro-2,4(1H,3H)-pyrimidinedione (1.60 mg, 3.4 mmol) in anhydrous tetrahydrofuran (4 ml) was gradually added to the solution, the temperataure was raised to -35° C and stirring was continued for 15 minutes. The reaction liquid was again cooled to -78° C. Thereafter triethylamine (2.4 ml, 17 mmol) was added thereto, the temperataure was raised to -35° C and stirring was continued for 15 minutes. The reaction liquid was cooled to -78° C and a solution of tri(t-butoxy)aluminum lithium hydride (4.35 g, 17 mmol) in anhydrous tetrahydrofuran (10 ml) was added thereto. While stirring, the temperature was slowly raised to room temperature. 0.2 M Phosphate buffer (pH 7.0) and methylene chloride were added to the reaction liquid. After insoluble matters were filtered off, extraction was performed with methylene chloride. After the extract was dried over anhydrous sodium sulfate, the solvent was distilled off under reduced pressure. The residue was purified by silica gel column chromatography (methylene chloride : ethyl acetate = 4 : 1 (v/v)) to give (±)-1-[(1β,2β,3β)-3-t-butyldiphenylsilyloxymethyl-2-hydroxycyclobutan-1-yl]-5-fluoro-2,4(1H,3H)-pyrimidinedione (628 mg, 39%).
NMR (400 MHz.FT, $CDCl_3$) δ:
1.07 (9H, s), 2.25-2.45 (3H, m), 3.21 (1H, d, J=4.0Hz), 3.85 (1H, dd, J=6.2Hz, J=11.0Hz), 3.94 (1H, dd, J=3.5Hz, J=11.0Hz), 4.71 (1H, m), 4.79 (1H, m), 7.32-7.50 (6H, m), 7.54 (1H, d, J=6.6Hz), 7.65 (4H, d, J=6.6Hz), 8.77 (1H, brs).

Step 2

Preparation of (±)-5-fluoro-1-[(1β,2β,3β)-2-hydroxy-3-hydroxymethylcyclobutan-1-yl]-2,4(1H,3H)-pyrimidinedione (Compound 10)

(±)-1-[(1β,2β,3β)-3-t-Butyldiphenylsilyloxymethyl-2-hydroxycyclobutan-1-yl]-5-fluoro-2,4(1H,3H)-pyrimidinedione (94 mg, 0.20 mmol) was dissolved in methanol (1 ml) and 4N-hydrochloric acid/dioxane (0.1 ml, 0.4 mmol) was added to the solution. The mixture was stirred at room temperature overnight. After the solvent was distilled off under reduced pressure, ether soluble matters were removed. Purification was performed by Sephadex LH-20 column chromatography (methanol) to give (±)-5-fluoro-1-[(1β,2β,3β)-2-hydroxy-3-hydroxymethylcyclobutan-1-yl]-2,4(1H,3H)-pyrimidinedione (Compound 10) (44 mg, 95%).
NMR (400 MHz.FT, $CD_3OD$) δ:
2.27-2.47 (3H, m), 3.62 (1H, dd, J=5.1HZ, J=11.0Hz), 3.80 (1H, dd, J=7.3Hz, J=11.0Hz), 4.58 (1H, m), 4.79 (1H, m), 4.91 (1H, d, J=7.0Hz).

Example 11

Preparation of (±)-1-[(1β,2β,3β)-2-hydroxy-3-hydroxymethylcyclobutan-1-yl]-5-methyl-2,4(1H,3H)-pyrimidinedione (Compound 11)

Step 1

Preparation of (±)-1-[(1β,2β,3β)-3-t-butyldiphenylsilyloxymethyl-2-hydroxycyclobutan-1-yl]-5-methyl-2,4-(1H,3H)pyrimidinedione

In an argon atmosphere, dimethylsulfoxide (341 μl, 4.8 mmol) was gradually added to a solution of oxalyl chloride (205 μl, 2.4 mmol) in tetrahydrofuran (5 ml) at -78° C. Then, the temperature was raised to -35° C and stirring was continued for 5 minutes at the same temperature. The reaction liquid was again cooled to -78° C. After a solution of (±)-1-[(1β,2α,3β)-3-t-butyldiphenylsilyloxymethyl-2-hydroxycyclobutan-1-yl]-5-methyl2,4(1H,3H)-pyrimidinedione (929 mg, 2.0 mmol) in anhydrous tetrahydrofuran (2 ml) was gradually added to the solution, the temperataure was raised to -35° C and stirring was continued for 15 minutes. The reaction liquid was again cooled to -78° C. Thereafter triethylamine (1.4 ml, 10 mmol) was added thereto, the temperataure was raised to -35° C and stirring was continued for 15 minutes. The reaction liquid was cooled to -78° C and a solution of tri(t-butoxy)aluminum lithium hydride (2.54 g, 10 mmol) in anhydrous tetrahydrofuran (10 ml) was added thereto. While stirring, the temperature was slowly raised to room temperature. 0.2 M Phosphate buffer (pH 7.0) and methylene chloride were added to the reaction liquid. After insoluble matters were filtered off, extraction was performed with methylene chloride. After the extract was dried over anhydrous sodium sulfate, the solvent was distilled off under reduced pressure. The residue was purified by silica gel column chromatography (methylene chloride : ethyl acetate = 4 : 1 (v/v)) to give (±)-1-[(1B,2B,3B)-3-t-butyldiphenylsilyloxymethyl-2-hydroxycyclobutan-1-yl]-5-methyl-2,4(1H,3H)pyrimidinedione (382 mg, 41%). NMR (400 MHz.FT, $CDCl_3$) δ:
1.08 (9H, s), 1.90 (3H, s), 2.32 (1H, m), 2.37-2.50 (2H, m), 3.32 (1H, m), 3.80-3.95 (2H, m), 4.68-4.77 (2H, m), 7.22 (1H, s), 7.35-7.50 (6H, m), 7.67 (4H, dd, J=1.8Hz, J=6.2Hz).

Step 2

Preparation of (±)-1-[(1β,2β,3β)-2-hydroxy-3-hydroxymethylcyclobutan-1-yl]-5-methyl-2,4(1H,3H)-pyrimidinedione (Compound 11)

(±)-1-[(1β,2β,3β)-3-t-Butyldiphenylsilyloxymethyl-2-hydroxycyclobutan-1-yl]-5-methyl-2,4(1H,3H)-pyrimidinedione (90 mg, 0.19 mmol) was dissolved in methanol (1 ml) and 4N-hydrochloric acid/dioxane (0.1 ml, 0.4 mmol) was added to the solution. The mixture was stirred at room temperature overnight. After the solvent was distilled off under reduced pressure, ether soluble matters were removed. Purification was performed by Sephadex LH-20 to give (±)-1-[(1β,2β,3β)-2-hydroxy-3-hydroxymethylcyclobutan-1-yl]-5-methyl-2,4(1H,3H)-pyrimidinedione (Compound 11) (43 mg, 98%)
NMR (400 MHZ.FT, $CD_3OD$) δ:
1.89 (3H, s), 2.29-2.48 (3H, m), 3.62 (1H, dd, J=4.6Hz, J=10.8Hz), 3.80 (1H, dd, J=6.8Hz, J=10.8Hz), 4.58 (1H, m), 4.71 (1H, m), 7.53 (1H, s).

Example 12

Preparation of (±)-1-[(1α,2β,3β)-2-hydroxy-3-hydroxymethylcyclobutan-1-yl]-2,4(1H,3H)-pyrimidinedione (Compound 12):

Step 1

Preparation of (±)-1-[(1α,2β,3β)-3-t-butyldiphenylsilyloxymethyl-2-hydroxycyclobutan-1-yl]-2,4(1H,3H)-pyrimidinedione

(±)-(1β,2β,3β)-2-t-Butyldiphenylsilyloxymethyl-5-oxabicyclo[2.1.0]pentane (Compound 1) (102 mg, 0.30 mmol) and 2,4-bistrimethylsilyloxypyrimidine (308 mg, 1.2 mmol) were dissolved in anhydrous methylene

chloride (3 ml) and an anhydrous methylene chloride solution (1 ml) of boron trifluoride etherate (51 mg, 0.36 mmol) was added to the solution at room temperature in an argon atmosphere. The mixture was stirred for a day as it was. To the reaction mixture was added 0.2 M phosphate buffer (pH 7.0). After stirring for a while, extraction was performed with methylene chloride. After the extract was dried over anhydrous sodium sulfate, the solvent was distilled off under reduced pressure. The residue was purified by silica gel column chromatography (methylene chloride : methanol = 50 : 1 (v/v)) to give (±)-1-[(1α,2β,3α)-3-t-butyldiphenylsilyloxymethyl-2-hydroxycyclobutan-1-yl]-2,4(1H,3H)-pyrimidinedione (88 mg, 65%).
NMR (400 MHz.FT, $CDCl_3$) δ:
1.10 (9H, s), 1.92 (1H, apparent q, J=10.3Hz), 2.26 (1H, apparent t, J=10.8Hz), 2.64 (1H, m), 3.70 (1H, d), 3.95 (1H, dd, J=4.8Hz, J=11.0Hz), 4.02 (1H,dd, J=5.3Hz, J=11.0Hz), 4.46 (1H, apparent q, J=7.3Hz), 4.68 (1H, apparent q, J=8.7Hz), 5.74 (1H, dd, J=1.8Hz, J=8.1Hz), 7.24 (1H, d, J=8.1Hz), 7.43 (6H, m), 7.69 (4H, apparent t, J=7.0Hz), 9.13 (1H, brs).

Step 2

Preparation of (±)-1-[(1α,2β,3β)-2-hydroxy-3-hydroxymethylcyclobutan-1-yl]-2,4(1H,3H)-pyrimidinedione (Compound 12)

(±)-1-[(1α,2β,3β)-3-t-Butyldiphenylsilyloxymethyl-2-hydroxycyclobutan-1-yl]-2,4(1H,3H)-pyrimidinedione (24 mg, 0.053 mmol) was dissolved in tetrahydrofuran (1 ml) and 1M-tetrabutyl ammonium/tetrahydrofuran (0.1 ml, 0.1 mmol) was added to the solution. The mixture was stirred at room temperature for 3 hours. Amberlyst 15 (60 mg, 0.3 mmol) was added to the reaction liquid. After stirring for a while,m the resin was filtered off and the solvent was removed by distillation. After the residue was purified by Sephadex LH-20 column chromatography (methanol), the residue was dissolved in water and the solution was freeze dried to give (±)-1-[(1α,2β,3β)-2-hydroxy-3-hydroxymethylcyclobutan-1-yl]-2,4(1H,3H)-pyrimidinedione (Compound 12) (12 mg, quantitative).
NMR (400 MHZ.FT, $CD_3OD$) δ:
1.94 (1H, apparent q, J=10.3Hz), 2.13 (1H, apparent t, J=10.5Hz), 2.57 (1H, m), 3.76 (1H, dd, J=8.4Hz, J=11.2Hz), 3.89 (1H, dd, J=5.6Hz, J=11.2Hz), 4.57 (1H, apparent t, J=8.4Hz), 4.79 (1H, apparent q, J=8.9Hz), 5.69 (1H, d, J=8.1Hz), 7.71 (1H, d, J=8.1Hz).

Example 13

Preparation of (±)-5-fluoro-1-[(1α,2β,3β)-2-hydroxy-3-hydroxymethylcyclobutan-1-yl]-2,4(1H,3H)-pyrimidinedione (Compound 13):

Step 1

Preparation of (±)-1-[(1α,2β,3β)-3-t-butyldiphenylsilyloxymethyl-2-hydroxycyclobutan-1-yl]-5-fluoro-2,4-(1H,3H)pyrimidinedione

(±)-(1β,2β,4β)-2-t-Butyldiphenylsilyloxymethyl-5-oxabicyclo[2.1.0]pentane (Compound 1) (169 mg, 0.50 mmol) and 2,4-bistrimethylsilyloxy-5-fluoropyrimidine (549 mg, 2.0 mmol) were dissolved in anhydrous methylene chloride (5 ml) and an anhydrous methylene chloride solution (1 ml) of boron trifluoride etherate (85 mg, 0.6 mmol) was added to the solution at room temperature in an argon atmosphere. The mixture was stirred for a day as it was. To the reaction mixture was added 0.2 M phosphate buffer (pH 7.0). After stirring for a while, extraction was performed with methylene chloride. After the extract was dried over anhydrous sodium sulfate, the solvent was distilled off under reduced pressure. The residue was purified by silica gel column chromatography (methylene chloride : methanol = 50 : 1 (v/v)) to give (±)-1-[(1α,2β,3β)-3-t-butyldiphenylsilyloxymethyl-2-hydroxycyclobutan-1-yl]-5-fluoro2,4(1H,3H)-pyrimidinedione (136 mg, 58%).
NMR (400 MHz.FT, $CDCl_3$) δ:
1.11 (9H, s), 1.89 (1H, apparent q, J=9.5Hz), 2.25 (1H, apparent t, J=10.5Hz), 2.63 (1H, m), 3.62 (1H, d,

J = 7.0Hz), 3.94 (1H, dd, J = 5.0Hz, J = 10.8Hz), 4.02 (1H, dd, J = 4.8Hz, J = 10.8Hz), 4.45 (1H, apparent q, J = 7.5Hz), 4.75 (1H, apparent q, J = 9.0Hz), 7.30 (1H, d, J = 5.9Hz), 7.33-7.50 (6H, m), 7.60-7.75 (4H, m), 8.90 (1H, brs).

Step 2

Preparation of (±)-5-fluoro-1-[(1α,2β,3β)-2-hydroxy-3-hydroxymethylcyclobutan-1-yl]-2,4(1H,3H)-pyrimidinedione (Compound 13)

(±)-1-[(1α,2β,3β)-3-t-Butyldiphenylsilyloxymethyl-2-hydroxycyclobutan-1-yl]-5-fluoro-2,4(1H,3H)-pyrimidinedione (123 mg, 0.26 mmol) was dissolved in methanol (2 ml) and 4N-hydrochloride/dioxane (0.15 ml, 0.6 mmol) was added to the solution. The mixture was stirred at room temperature overnight. After the solvent was removed by distillation, ether soluble matters were removed. After the residue was purified by Sephadex LH-20 (methanol), the residue was dissolved in water and the solution was freeze dried to give (±)-5-fluoro-1-[(1α,2β,3β)-2-hydroxy-3-hydroxymethylcyclobutan-1-yl]-2,4(1H,3H)pyrimidinedione (Compound 13) (48 mg, 79%).
NMR (400 MHz.FT, $CD_3OD$) δ:
1.93 (1H, apparent q, J = 10.6Hz), 2.12 (1H, apparent t, J = 10.3Hz), 2.56 (1H, m), 3.76 (1H, dd, J = 7.5Hz, J = 11.2Hz), 3.88 (1H, dd, J = 5.9Hz, J = 11.2Hz), 4.54 (1H, apparent t, J = 8.2Hz), 4.84 (1H, m), 7.97 (1H, d, J = 6.6Hz).

Example 14

Preparation of (±)-1-[(1β,2α,3β)-2-azido-3-hydroxymethylcyclobutan-1-yl]-5-fluoro-2,4(1H,3H)-pyrimidinedione (Compound 14)

Step 1

Preparation of (±)-1-[(1β,2β,3β)-3-t-butyldiphenylsilyloxymethyl-2-methanesulfonyloxycyclobutan-1-yl]-5-fluoro-2,4(1H,3H)-pyrimidinedione

Methanesulfonyl chloride (23 µl, 0.30 mmol) was added to a solution of (±)-1-[(1β,2β,3β)-3-t-butyldiphenylsilyloxymethyl-2-hydroxycyclobutan-1-yl]-5-fluoro-2,4(1H,3H)-pyrimidinedione (94 mg, 0.20 mmol) and triethylamine (50.µl, 0.36 mmol) under ice cooling. The mixture was stirred at the same temperataure for 30 minutes. Further triethylamine (83 µl, 0.60 mmol) and methanesulfonyl chloride (23 µl, 0.30 mmol) were added thereto followed by stirring for an hour. To the reaction liquid was added 0.2 M phosphate buffer (pH 7.0) and extraction was performed with methylene chloride. After the extract was dried over anhydrous sodium sulfate, the solvent was distilled off under reduced pressure. The residue was purified by silica gel column chromatography (methylene chloride : ethyl acetate = 20 : 1 (v/v)) to give (±)-1-[(1β,2β,3β)-3-t-butyldiphenylsilyloxymethyl-2-methanesulfonyloxycyclobutan-1-yl]-5-fluoro-2,4(1H,3H)-pyrimidinedione (36 mg, 33%).

Step 2

Preparation of (±)-1-[(1β,2α,3β)-2-azido-3-t-butyldiphenylsilyloxymethylcyclobutan-1-yl]-5-fluoro-2,4(1H,3H)-pyrimidinedione and (±)-1-[(1β,2α,3β)-2-azido-3-hydroxymethylcyclobutan-1-yl]-5-fluoro-2,4(1H,3H)-pyrimidinedione (Compound 14)

In an argon atmosphere, a solution of (±)-1-[(1β,2β,3β)-3-t-butyldiphenylsilyloxymethyl-2-methanesulfonyloxycyclobutan-1-yl]-5-fluoro-2,4(1H,3H)-pyrimidinedione (75 mg, 0.14 mmol) and dry sodium azide (90 mg, 1.4 mmol) in anhydrous dimethylformamide (2 ml) was stirred at 110 °C for 7 hours. Water was added to the reaction liquid followed by extraction with methylene chloride. After the methylene chloride extract was dried over anhydrous sodium sulfate, the solvent was distilled off under reduced pressure. The residue was purified by silica gel column chromatography (methylene chloride : methanol = 15 : 1 (v/v)) to give (±)-1-[(1β,2α,3β)-2-azido-3-t-butyldiphenylsilyloxymethylcyclobutan-1-yl]-5-fluoro-2,4-(1H,3H)-pyrimidinedione (15 mg, 22%).

NMR (400 MHZ.FT, $CDCl_3$) δ:

1.11 (9H, s), 1.97 (1H, m), 2.22-2.35 (2H, m), 3.72 (1H, dd, J=3.3Hz, J=11.4Hz), 3.87 (1H, dd, J=2.6Hz, J=11.4Hz), 3.95 (1H, apparent t, J=7.7Hz), 4.68 (1H, m), 7.30 (1H, d, J=5.5Hz), 7.32-7.50 (6H, m), 7.57-7.72 (4H, m), 8.72 (1H, br).

The aqueous phase which was previously subjected to extraction was further continuously extracted with methylene chloride for a day. The methylene chloride extract was purified by silica gel preparative thin layer chromatography (methylene chloride : methanol = 15 : 1 (v/v)) to give (±)-1-[(1β,2α,3β)-2-azido-3-hydroxymethylcyclobutan-1-yl]-5-fluoro-2,4(1H,3H)pyrimidinedione (Compound 14) (4.8 mg, 14%).

NMR (400 MHz.FT, $CDCl_3$) δ:

2.03 (1H, apparent q, J=9.6Hz), 2.26-2.45 (2H, m), 3.74 (1H, dd, J=3.8Hz, J=11.0Hz), 3.87 (1H, dd, J=3.3Hz, J=11.0Hz), 4.55 (1H, brs), 4.57-4.70 (2H, m), 7.40 (1H, d, J=5.9Hz), 8.23 (1H, brd).

IR(KBr)$cm^{-1}$: 2110.

Example 15

Preparation of (±)-1-[(1β,2α,3β)-2-azido-3-hydroxymethylcyclobutan-1-yl]-5-methyl-2,4(1H,3H)-pyrimidinedione (Compound 15):

Step 1

Preparation of (±)-1-[(1β,2β,3β)-3-t-butyldiphenylsilyloxymethyl-2-methanesulfonyloxycyclobutan-1-yl]-5-methyl-2,4(1H,3H)-pyrimidinedione

Under ice cooling, a solution of methanesulfonyl chloride (150 mg, 1.3 mmol) in methylene chloride (1 ml) was added to a solution of (±)-(1β,2β,3β)-3-t-Butyldiphenylsilyloxymethyl-2-hydroxyclobutan-1-yl]-5-methyl-2,4(1H,3H)pyrimidinedione (300 mg, 0.65 mmol) and triethylamine (0.5 ml, 3.6 mmol) in anhydrous methylene chloride (5 ml). The mixture was stirred at the same temperature for 30 minutes. Further, the same amounts of triethylamine and methanesulfonyl chloride were added to the mixture followed by stirring for an hour. To the reaction mixture was added 0.2 M phosphate buffer (pH 7.0). The mixture was extracted with methylene chloride. After the extract was dried over anhydrous sodium sulfate, the solvent was distilled off under reduced pressure. The residue was purified by silica gel column chromatography (methylene chloride : ethyl acetate = 3 : 1 (v/v)) to give (±)-1-[(1β,2β,3β)-3-t-butyldiphenylsilyloxymethyl-2-methanesulfonyloxycyclobutan-1-yl]-5-methyl-2,4(1H,3H)-pyrimidinedione (184 mg, 53%).

Step 2

Preparation of (±)-1-[(1β,2α,3β)-2-azido-3-t-butyldiphenylsilyloxymethylcyclobutan-1-yl]-5-methyl-2,4(1H,3H)-pyrimidinedione

In an argon atmosphere, a solution of (±)-1-[(1β,2β,3β)-3-t-butyldiphenylsilyloxymethyl-2-methanesulfonyloxycyclobutan-1-yl]-5-methyl-2,4(1H,3H)-pyrimidinedione (25 mg, 46 µmol) and dry sodium azide (15 mg, 230 µmols) in anhydrous dimethylformamide (1 ml) was stirred at 110 °C for 10 hours. Water was added to the reaction liquid and the mixture was extracted with ethyl acetate. After the ethyl acetate extract was dried over anhydrous sodium sulfate, the solvent was removed by distillation. The residue was purified by silica gel column chromatography (methylene chloride : ethyl acetate = 10 : 1 (v/v)) to give (±)-1-[(1β,2α,3β)-2-azido-3-t-butyldiphenylsilyloxymethylcyclobutan-1-yl]-5-methyl-2,4(1H,3H)-pyrimidinedione

(16 mg, 71%).
NMR (400 MHz.FT, $CDCl_3$) δ:
1.12 (9H, s), 1.90 (3H, s), 2.04 (1H, m), 2.18-2.34 (2H, m), 3.72 (1H, dd, J=2.9Hz, J=11.4Hz), 3.83 (1H, dd, J=2.2Hz, J=11.4Hz), 4.03 (1H, t, J=7.7Hz), 4.75 (1H, q, J=8.8Hz), 7.08 (1H, s), 7.33-7.51 (6H, m), 7.65 (4H. m), 8.72 (1H, br).

Step 3

Preparation of (±)-1-[(1β,2α,3β)-2-azido-3-hydroxymethylcyclobutan-1-yl]-5-methyl-2,4(1H,3H)-pyrimidinedione (Compound 15)

(±)-1-[(1β,2α,3β)-2-Azido-3-t-butyldiphenylsilyloxymethylcyclobutan-1-yl]-5-methyl-2,4(1H,3H)-pyrimidinedione (111 mg, 0.23 mmol) was dissolved in methanol (1 ml) and 4N-hydrochloric acid/dioxane (0.15 ml, 0.6 mmol) was added to the solution. The mixture was stirred at room temperature overnight. After the solvent was distilled off under reduced pressure, ether soluble matters were removed. Purification was performed by Sephadex LH-20 column chromatography (methanol) to give (±)-1-[(1β,2α,3β)-2-azido-3-hydroxymethylcyclobutan-1-yl]-5-methyl-2,4(1H,3H)-pyrimidinedione (Compound 15) (58 mg, quantitative).
NMR (400 MHz.FT, $CDCl_3$) δ:
1.94 (3H, s), 2.08 (1H, m), 2.22-2.40 (3H, m), 3.74 (1H, dd, J=4.0Hz, J=11.4Hz), 3.86 (1H, dd, J=3.3Hz, J=11.4Hz), 4.14 (1H, apparent t, J=7.7Hz), 4.55 (1H, apparent q, J=8.9Hz), 7.11 (1H, s), 9.09 (1H, br).
IR(neat) $cm^{-1}$: 2120.

Example 16

Preparation of (±)-1-[(1β,2α,3β)-2-amino-3-hydroxymethylcyclobutan-1-yl]-5-fluoro-2,4(1H,3H)-pyrimidinedione (Compound 16):

Step 1

Preparation of (±)-1-[(1β,2α,3β)-2-amino-3-t-butyldiphenylsilyloxymethylcyclobutan-1-yl]-5-fluoro-2,4(1H,3H)-pyrimidinedione

(±)-1-[(1β,2α,3β)-2-Azido-3-t-butyldiphenylsilyloxymethylcyclobutan-1-yl]-5-fluoro-2,4(1H,3H)-pyrimidinedione (14 mg, 28 μmol) was dissolved in methanol (1 ml) and 10% palladium on carbon (3 mg) was added to the solution. The mixture was stirred for 24 hours under hydrogen atmosphere. After the catalyst was filtered off, the solvent was distilled off under reduced pressure. The residue was purified by silica gel column chromatography (methylene chloride : methanol = 20 : 1 (v/v)) to give (±)-1-[(1β,2α,3β)-2-amino-3-t-butyldiphenylsilyloxymethylcyclobutan-1-yl]-5-fluoro-2,4(1H,3H)-pyrimidinedione (7.2 mg, 54%).
NMR (400 MHz.FT, $CDCl_3$) δ:
1.10 (9H, s), 1.72 (1H, apparent q, J=10.1Hz), 1.90 (1H, m), 2.20 (1H, apparent q, J=8.9Hz), 2.75-3.50 (2H, br), 3.36 (1H, apparent t, J=8.3Hz), 3.72 (1H, dd, J=3.8Hz, J=11.0Hz), 3.83 (1H, dd, J=3 3Hz, J=11.0Hz), 4.42 (1H, m), 7.30-7.50 (6H, m),7.35 (1H, d, J=5.9Hz), 7.50-7.75 (4H, m).

Step 2

Preparation of (±)-1-[(1β,2α,3β)-2-amino-3-hydroxymethylcyclobutan-1-yl]-5-fluoro-2,4(1H,3H)-pyrimidinedione (Compound 16)

(±)-1-[(1β,2α,3β)-2-Amino-3-t-butyldiphenylsilyloxymethylcyclobutan-1-yl]-5-fluoro-2,4(1H,3H)-pyrimidinedione (7 mg, 15 μmol) was dissolved in methanol (1 ml) and 4N-hydrochloric acid/dioxane (0.1

ml, 0.4 mmol) was added to the solution. The mixture was stirred at room temperature overnight. After the solvent was distilled off under reduced pressure, ether-soluble matters were removed and the residue was purified by Sephadex LH-20 column chromatography (methanol) to give (±)-1-[(1β,2α,3β)-2-amino-3-hydroxymethylcyclobutan-1-yl-5-fluoro-2,4(1H,3H)-pyrimidinedione hydrochloride (Compound 16) (4 mg, quantitative).
NMR (400 MHz.FT, $CD_3OD$) δ:
2.10 (1H, apparent q, J=9.6Hz), 2.30-2.52 (2H, m), 3.56-3.78 (3H, m), 4.56 (1H, apparent q, J=8.5Hz), 7.79 (1H, d, J=6.3Hz).

Example 17

Preparation of (±)-1-[(1β,2α,3β)-2-amino-3-hydroxymethylcyclobutan-1-yl]-5-methyl-2,4(1H,3H)-pyrimidinedione (Compound 17):

Step 1

Preparation of (±)-1-[(1β,2α,3β)-2-amino-3-t-butyldiphenylsilyloxymethylcyclobutan-1-yl]-5-methyl-2,4-(1H,3H)pyrimidinedione

(±)-(1β,2α,3β)-2-Azido-3-t-butyldiphenylsilyloxymethylcyclobutan-1-yl]-5-methyl-2,4(1H,3H)-pyrimidinedione (12 mg, 25 µmol) was dissolved in methanol (2 ml) and 10% palladium on carbon (2 mg) was added to the solution. The mixture was stirred for 10 hours under hydrogen atomosphere. After the solvent was distilled off under reduced pressure, ether soluble matters were removed and the residue was purified by Sephadex LH-20 column chromatography (methanol) to give (±)-1-[(1β,2α,3β)-2-amino-3-t-butyldiphenylsilyloxymethylcyclobutan-1-yl]-5-methyl-2,4(1H,3H)-pyrimidinedione (10 mg, 88%).
NMR (400 MHz.FT, $CDCl_3$) δ:
1.08 (9H, s), 1.90 (3H, s), 2.32 (1H, m), 2.38-2.52 (2H, m), 3.32 (1H, m), 3.85-3.95 (2H, m), 4.66-4.78 (2H, m), 7.22 (1H, s), 7.33-7.49 (6H, m), 7.67 (4H, dd, J=1.8Hz, J=6.2Hz)

Step 2

Preparation of (±)-1-[(1β,2α,3β)-2-amino-3-hydroxymethylcyclobutan-1-yl]-5-methyl-2,4(1H,3H)-pyrimidinedione (Compound 17)

(±)-1-[(1β,2α,3β)-2-Amino-3-t-butyldiphenylsilyloxymethylcyclobutan-1-yl]-5-methyl-2,4(1H,3H)-pyrimidinedione (10 mg, 22 µmol) was dissolved in methanol (1 ml) and 4N-hydrochloric acid/dioxane (0.1 ml, 0.4 mmol) was added to the solution. The mixture was stirred at room temperature overnight. After the solvent was distilled off under reduced pressure, ether soluble matters were removed and the residue was purified by Sephadex LH-20 column chromatography (methanol) to give (±)-1-[(1β,2α,3β)-2-amino-3-hydroxymethylcyclobutan-1-yl-5-methyl-2,4(1H,3H)-pyrimidinedione hydrochloride (Compound 17) (7 mg, quantitative).
NMR (400 MHz.FT, $CD_3OD$) δ:
1.94 (3H, s), 2.17 (1H, q, J=9.0Hz), 2.40-2.60 (2H, m), 3.63 (1H, m), 3.65-3.85 (2H, m), 4.57 (1H, q, J=8.5Hz), 7.43 (1H, s).

Example 18

Preparation of (±)-(1α,2β,4α)-2-t-butyldiphenylsilyloxymethyl-5-oxabicyclo[2.1.0]pentane (Compound e) and (±)-1-[(1β,2β,4β)-2-t-butyldiphenylsilyloxymethyl-5-oxabicyclo[2.1.0]pentane (Compound 1):

1-t-Butyldiphenylsilyloxymethyl-2-cyclobutene (6.45 g, 20 mmol) and 2-benzenesulfonyl-3-(4-nitrophenyl)oxazylidine (XIII) (F.A. Davis et al., Tetrahedron Lett., 22, 917 (1981)) (7.35 g, 24 mmol) were added to anhydrous acetonitrile (100 ml). The mixture was stirred at 60° C for 4 days in an argon atmosphere. After cooling, acetonitrile was distilled off under reduced pressure and the residue was extracted with hexane. The hexane extract was concentrated and the concentrate was separated and purified by silica gel column chromatography (methylene chloride : hexane = 1 : 3 and then 1 : 2 (v/v)) to give the unreacted starting material (2.15 g, 33% recovery), (±)-(1α,2β,4α)-2-t-butyldiphenylsilyloxymethyl-5-oxabicyclo [2.1.0]pentane (Compound e) and (±)-1-[(1β,2β,4β)-2-t-butyldiphenylsilyloxymethyl-5-oxabicyclo-[2.1.0]pentane (Compound 1) (0.71 g, 10%).

(Compound e)

NMR (400 MHz.FT, $CDCl_3$) δ:
1.07 (9H, s), 1.66 (1H, dd, J=3.7Hz, J=12.1Hz), 1.83 (1H, ddd, J=2.6Hz, J=5.1Hz, J=12.1Hz), 2.32 (1H, m), 3.70 (1H, dd, J=8.4Hz, J=10.6Hz), 3.79 (1H, m), 3.82-3.88 (2H, m), 7.34-7.47 (6H, m), 7.60-7.72 (4H, m).

(Compound 1)

NMR (400 MHz.FT, $CDCl_3$) δ:
1.06 (9H, s), 1.36 (1H, dt, J=12.1Hz, J=3.6Hz), 2.08 (1H, dd, J=9.2Hz, J=12.1Hz), 2.63 (1H, m), 3.45 (1H, dd, J=6.4Hz, J=10.4Hz), 3.72-3.82 (2H, m), 3.84 (1H, m), 7.33-7.45 (6H, m), 7.67 (4H, apparent t, J=7.75Hz).

Example 19

Preparation of (±)-(1α,2β,4α)-2-benzoyloxymethyl-5-oxabicyclo[2.1.0]pentane (Compound c) and (±)-1-[-(1β,2β,4β)-2-benzoyloxymethyl-5-oxabicyclo[2.1.0]pentane (Compound j)

Method 1

1-t-Butyldimethylsilyloxymethyl-2-cyclobutene (1.0 g, 5.0 mmol) and 2-benzenesulfonyl-3-(4-nitrophenyl)oxazylidine (XIII) (2.3 g, 7.5 mmol) were added to anhydrous acetonitrile 25 ml). The mixture was stirred at 60° C for 2 days in an argon atmosphere. After cooling, acetonitrile was distilled off under reduced pressure and the residue was extracted with pentane. The solvent was removed from the pentane extract by distillation under reduced pressure. The residue was dissolved in anhydrous tetrahydrofuran (25 ml) and 1M-tetrabutyl ammonium fluoride/tetrahydrofuran (6.0 ml, 6.0 mmol) was added to the solution. The mixture was stirred at room temperature for an hour. To the reaction mixture was added 0.2 M phosphate buffer (pH 7.0). After the mixture was saturated with sodium salt, extraction was performed with a solvent mixture of methylene chloride-methanol (4 : 1 (v/v)) 10 times. After the extract was dried over anhydrous sodium sulfate, the solvent was distilled off under reduced pressure. The residue was purified by silica gel column chromatography (ethyl acetate : hexane = 1 : 1 (v/v)) to give 210 mg (42%) of 2-hydroxymethyl-5-oxabicyclo[2.1.0]pentane (mixture of Compound a and Compound h). This compound was dissolved in absolute pyridine (2 ml) and benzoyl chloride (0.37 ml, 3.2 mmol) was added to the solution under ice cooling. The mixture was stirred for an hour at the same temperature. Water was added to the reaction liquid and the mixture was extracted with ether. After the ethereal extract was washed with 1M sulfuric acid aqueous solution, water, aqueous sodium chloride solution in sequence, the extract was dried over anhydrous sodium sulfate. The solvent was distilled off under reduced pressure to give oil. The oil was purified by silica gel column chromatography (methylene chloride : hexane = 1 : 1 (v/v)) to give (±)-(1α,2β,4α)-2-benzoyloxymethyl-5-oxabicyclo [2.1.0]pentane (Compound c) (268 mg, overall yield 26%) and (±)-1-[(1β,2β,4β)-2-benzoyloxymethyl-5-oxabicyclo[2.1.0]pentane (Compound j) (76 mg, overall yield 7%).

(Compound c)

NMR (400 MHz.FT, $CDCl_3$) δ:
1.82 (1H, ddd, J = 1.8Hz, J = 4.6Hz, J = 12.0Hz), 2.04 (1H, ddd, J = 2.8Hz, J = 5.6Hz, J = 12.0Hz), 2.55 (1H, m), 3.91 (1H, m), 3.97 (1H, t, J = 2.8Hz), 4.39 (1H, dd, J = 9.0Hz, J = 11.7Hz), 4.55 (1H, dd, J = 6.0Hz, J = 11.7Hz), 7.46 (2H, t, J = 8.1Hz), 7.59 (1H, apparent t, J = 8.1Hz), 8.05 (1H, apparent d, J = 8.1Hz).

(Compound j)

NMR (400 MHz.FT, $CDCl_3$) δ:
1.62 (1H, dt, J = 12.1Hz, J = 3.6Hz), 2.29 (1H, dd, J = 9.5Hz, J = 12.1Hz), 2.86 (1H, m), 3.88 (1H, m), 3.92 (1H, m), 4.21 (1H, dd, J = 6.5Hz, J = 12.1Hz), 4.39 (1H, dd, J = 8.9Hz, J = 12.1Hz), 7.44 (2H, t, J = 8.2Hz), 7.56 (1H, apparent t, J = 8.2Hz), 8.04 (1H, apparent d, J = 8.2Hz).

Method 2

1-Hydroxymethyl-2-cyclobutene (252 mg, 3.0 mmol) was dissolved in anhydrous methylene chloride (15 ml) and 80% m-chloroperbenzoic acid (777 mg, 3.6 mmol) was added to the solution. The mixture was stirred for 3 hours at 0° C. To the reaction liquid were added 10% sodium hydrogensulfite aqueous solution and 10% potassium carbonate aqueous solution. The mixture was extracted with methylene chloride 10 times. After the extract was dried over anhydrous sodium sulfate, the solvent was distilled off under reduced pressure to give the crude product. The crude product was benzoylated using benzoyl chloride (0.52 ml, 4.5 mmol) and anhydrous pyridine (3 ml) in accordance with Method 1 and then separated and purified to give (±)-(1α,2β,4α)-2-benzoyloxymethyl-5-oxabicyclo[2.1.0]pentane (Compound c) (67 mg, 11%) and (±)-1-[-(1β,2β,4β)-2-benzoyloxymethyl-5-oxabicyclo[2.1.0]pentane (Compound j) (180 mg, 29%).

Example 20

Preparation of 1-[(1β,3β)-3-hydroxymethylcyclobutan-1-yl]-5-methyl-2,4(1H,3H)-pyrimidinedione (Compound 3-21):

Step 1

Preparation of (1β,3β)-3-t-butyldiphenylsilyloxymethylcyclobutylazide

Sodium azide (650 mg, 10 mmol) and (1β,3β)-3-t-butyldiphenylsilyloxymethyl-1-methanesulfonyloxycyclobutane (419 mg, 1.0 mmol) were dissolved in dimethylformamide (6 ml) in an argon atmosphere. The mixture was stirred at 120° C for 2 hours. Water was added to the reaction liquid and the mixture was extracted with ether. The ethereal extract was washed with water twice and then washed with saturated sodium chloride aqueous solution. After the ethereal solution was dried over anhydrous sodium sulfate, the solvent was distilled off under reduced pressure and the residue was purified by silica gel column chromatography (hexane : methylene chloride = 4 : 1 (v/v)) to give (1β,3β)-3-t-butyldiphenylsilyloxymethylcyclobutylazide (309 mg, 81%).

Step 2

Preparation of (1β,3β)-3-t-butyldiphenylsilyloxymethylcyclobutylamine

(1β,3β)-3-t-Butyldiphenylsilyloxymethylcyclobutylazide (298 mg, 0.78 mmol) was dissolved in ethanol (2 ml) and 10% palladium on carbon (20 mg) was added to the solution. The mixture was stirred at room temperature for an hour under hydrogen atmosphere. After the catalyst was removed by filtration, the solvent was distilled off under reduced pressure and the residue was purified by silica gel column chromatography (ethyl acetate : triethylamine = 20 : 1 (v/v)) to give (1β,3β)-3-t-butyldiphenylsilyloxymethylcyclobutylamine (231 mg, 83%).

Step 3

Preparation of N-[[(1β,3β)-3-t-butyldiphenylsilyloxymethylcyclobutyl]aminocarbonyl]-3-methoxy-2-methylacrylamide

A suspension of 3-methoxy-2-methylacryloyl chloride (673 mg, 5 mmol) and silver cyanate (1.50 g, 10 mmol) in anhydrous benzene (10 ml) was heated to reflux for an hour in an argon atmosphere and then allowed to stand at room temperature. The supernatant (1.7 ml) of the mixture was taken out and added to a solution of (1β,3β)-3-t-butyldiphenylsilyloxymethylcyclobutylamine (230 mg, 0.68 mmol) in anhydrous benzene at 0°C. The mixture was stirred overnight as it was. After the solvent was distilled off under reduced pressure, the residue was purified by silica gel column chromatography (hexane : ethyl acetate = 3 : 1 (v v)) to give N-[[(1β,3β)-3-t-butyldiphenylsilyloxymethylcyclobutyl]aminocarbonyl]-3-methoxy-2-methylacrylamide (132 mg, 41%).

Step 4

Preparation of 1-[(1β,3β)-3-hydroxymethylcyclobutan-1-yl]-5-methyl-2,4(1H,3H)-pyrimidinedione (Compound 3-21)

N-[[(1β,3β)-3-t-Butyldiphenylsilyloxymethylcyclobutyl]aminocarbonyl]-3-methoxy-2-methylacrylamide (126 mg, 0.26 mmol) was dissolved in methanol (2 ml) and 4N-hydrochloric acid/dioxane (0.1 ml, 4 mmol) was added to the solution. The mixture was stirred at room temperature overnight. After the solvent was distilled off under reduced pressure, water was added thereto and ether soluble matters were removed. The solvent was distilled off under reduced pressure. 2N-Sulfuric acid (1.2 ml) was added to the residue and the mixture was heated to reflux for 30 minutes. The reaction liquid was purified by Sephadex HP20 (water-50% methanol aqueous solution) to give 1-[(1β,3β)-3-hydroxymethylcyclobutan-1-yl]-5-methyl-2,4(1H,3H)-pyrimidinedione (33 mg, 51%).
NMR (200 MHz.FT, $CD_3OD$) δ:
1.90 (3H, d, J=1.2Hz), 2.00-2.18.(2H, m), 2.26 (1H, m), 2.32-2.50 (2H, m), 3.57 (2H, d, J=5.0Hz), 4.74 (1H, m), 7.51 (1H, J=1.2Hz).
UV $\lambda_{max}(H_2O)$ nm:
pH 1, 273; pH 7, 273; pH 13, 271.

Example 21

Preparation of 1-[(1β,3β)-3-hydroxymethylcyclobutan-1-yl]-2,4(1H,3H)-pyrimidinedione (Compound 3-19):

Step 1

Preparation of N-[[(1β,3β)-3-t-butyldiphenylsilyloxymethylcyclobutyl]aminocarbonyl]-3-ethoxyacrylamide

A suspension of 3-ethoxyacryloyl chloride (673 mg, 5 mmol) and silver cyanate (1.50 g, 10 mmol) in anhydrous benzene (10 ml) was heated to reflux for an hour in an argon atmosphere and then allowed to stand at room temperature. The supernatant (1.7 ml) of the mixture was taken out and added to a solution of (1β,3β)-3-t-butyldiphenylsilyloxymethylcyclobutylamine (cf. Step 2 in Example 20) (219 mg, 0.64 mmol) in anhydrous benzene at 0°C. The mixture was stirred overnight as it was. After the solvent was distilled off under reduced pressure, the residue was purified by silica gel column chromatography (hexane : ethyl acetate = 3 : 1 (v/v)) to give
N-[[(1β,3β)-3-t-butyldiphenylsilyloxymethylcyclobutyl]aminocarbon yl]-3-ethoxyacrylamide (194 mg, 63%).

Step 2

Preparation of 1-[(1β,3β)-3-hydroxymethylcyclobutan-1-yl]-2,4(1H,3H)-pyrimidinedione (Compound 3-19)

N-[[(1β,3β)-3-t-Butyldiphenylsilyloxymethylcyclobutyl]aminocarbonyl]-3-ethoxyacrylamide (194 mg, 0.40 mmol) was dissolved in methanol (2 ml) and 1N-tetrabutyl ammonium fluoride/tetrahydrofuran (0.6 ml, 4 mmol) was added to the solution. The mixture was stirred at room temperature for 4 hours. After the solvent was distilled off under reduced pressure, water was added thereto and ether soluble matters were removed. The solvent was distilled off under reduced pressure. 2N-Sulfuric acid (2 ml) was added to the residue and the mixture was heated to reflux for 2 hours. The reaction liquid was purified by Sephadex HP20 (water-20% methanol aqueous solution) and further purified by preparative high performance liquid chromatography (ODS, water : acetonitrile = 90 : 10 (v/v)) to give 1-[(1β,3β)-3-hydroxymethylcyclobutan-1-yl]-2,4-(1H,3H)-pyrimidinedione (12 mg, 14%).
NMR (200 MHz.FT, $CD_3OD$) δ:
1.97-2.17 (3H, m), 2.28 (1H, m), 2.34-2.52 (2H, m), 3.56 (2H, d, J=5.1Hz), 4.68 (1H, m), 5.68 (1H, d, J=8.0Hz), 7.72 (1H, d, J=8.0Hz).
UV $\lambda_{max}(H_2O)$ nm:
pH 1, 272; pH 7, 272; pH 13, 270.

Example 22

Preparation of (±)-2-amino-6-chloro-9-[(1β,2α,3β)-2-hydroxy-3-hydroxymethylcyclobutan-1-yl]purine (Compound 4-3):

Step 1

Preparation of (±)-9-[(1β,2α,3β)-2-acetoxy-3-acetoxymethylcyclobutan-1-yl]-2-amino-6-chloropurine

Pyridine (0.5 ml) and acetic anhydride acid (1 ml) were added to a suspension of (±)-9-[(1β,2α,3β)-2-hydroxy-3-hydroxymethylcyclobutan-1-yl]guanine (100 mg, 0.4 mmol) in anhydrous dimethylformamide (1.5 ml). The mixture was stirred at 50° C for 15 minutes. After the solvent was distilled off under reduced pressure and azeotropically distilled with toluene several times, the mixture was dried at 40° C in vacuum to give crude (±)-9-[(1β,2α,3β)-2-acetoxy-3-acetoxymethylcyclobutan-1-yl]guanine (135 mg).

The thus obtained crude (±)-9-[(1β,2α,3β)-2-acetoxy-3-acetoxymethylcyclobutan-1-yl]guanine (135 mg) and tetraethyl ammonium chloride (133 mg, 0.8 mmol) were dissolved in anhydrous acetonitrile (1 ml) and, N,N-dimethylaniline (51 μl, 0.4 mmol) and phosphorus oxychloride (0.3 ml, 3.2 mmol) were added to the solution. The mixture was heated to reflux at 100° C for 10 minutes. Volatile substances were removed from the reaction liquid by distillation under reduced pressure. The residue was dissolved in methylene chloride. Immediately thereafter crushed ice was added to the methylene chloride solution. To the methylene chloride extract were added water and crushed ice. After stirring for a while, the system was rendered alkaline with ammonia water. This mixture was further extracted with methylene chloride. The extract was washed with 0.2 M phosphate buffer. After drying over anhydrous sodium sulfate, the solvent was distilled off. The residue was purified by silica gel column chromatography (ethyl acetate) to give (±)-9-[(1β,2α,3β)-2-acetoxy-3-acetoxymethylcyclobutan-1-yl]-2-amino- 6-chloropurine (89 mg, 63%).
NMR (200 MHz.FT, $CDCl_3$) δ:
2.02 (3H, s), 2.09 (3H, s), 2.25 (1H, m), 2.40-2.64 (2H, m), 4.27 (1H, dd, J=5.5Hz, J=11.6Hz), 4.37 (1H, dd, J=5.1Hz, J=11.6Hz), 4.66 (1H, apparent q, J=8.1Hz), 5.06 (2H, brs), 5.50 (1H, apparent t, J=7.1Hz), 7.83 (1H, s).

Step 2

Preparation of (±)-2-amino-6-chloro-9-[(1β,2α,3β)-2-hydroxy-3-hydroxymethylcyclobutan-1-yl]purine (Compound 4-3)

(±)-9-[(1β,2α,3β)-2-Acetoxy-3-acetoxymethylcyclobutan-1-yl]-2-amino-6-chloropurine (22 mg, 62 µmol) was dissolved in methanol (1 ml) and potassium carbonate (20 mg, 140 µmol) was added to the solution followed by stirring at 0° C for 30 minutes. After neutralizing with 0.1N-hydrochloric acid, the reaction liquid was purified by Sephadex HP20 (water-50% methanol aqueous solution) to give (±)-2-amino-6-chloro-9-[-(1β,2α,3β)-2-hydroxy-3-hydroxymethylcyclobutan-1-yl]purine (14 mg, 85%).
NMR (400 MHz.FT, $CD_3OD$) δ:
2.01 (1H, m), 2.18 (1H, m), 2.38 (1H, m), 3.70-3.80 (2H, m), 4.45-4.57 (2H, m), 8.15 (1H, s). UV $\lambda_{max}(H_2O)$ nm:
pH 1, 244(sh), 313; pH 7, 245, 304; pH 13, 247(sh), 307.

Example 23

Preparation of (±)-2,6-diamino-9-[(1β,2α,3β)-2-hydroxy-3-hydroxymethylcyclobutan-1-yl]purine (Compound 4-4):

(±)-9-[(1β,2α,3β)-2-Acetoxy-3-acetoxymethylcyclobutan-1-yl]-2-amino-6-chloropurine (13 mg, 37 µmol) was dissolved in ethanol (1 ml) and the solution was cooled to -78° C. The solution was saturated with liquid ammonia and heated at 110° C for 24 hours in a sealed tube. Volatile substances were distilled off under reduced pressure and the remaining matter was dissolved in water. After neutralizing with 0.1N-hydrochloric acid, the solution was purified by Sephadex HP20 (water-50% methanol aqueous solution) to give (±)-2,6-diamino-9-[(1β,2α,3β)-2-hydroxy-3-hydroxymethylcyclobutan-1-yl]purine (9 mg, 98%).
NMR (400 MHz.FT, $CD_3OD$) δ:
1.89 (1H, m), 2.18 (1H, m), 2.39 (1H, m), 3.70-3.80 (2H, m), 4.37-4.50 (2H, m), 7.86 (1H, s).
UV $\lambda_{max}(H_2O)$ nm:
pH 1, 254, 291; pH 7, 255, 280; pH 13, 256, 281.

Example 24

Preparation of (±)-2-amino-9-[(1β,2α,3β)-2-hydroxy-3-hydroxymethylcyclobutan-1-yl]purine (Compound 4-1)

Step 1

Preparation of (±)-9-[(1β,2α,3β)-2-acetoxy-3-acetoxymethylcyclobutan-1-yl]-2-aminopurine

(±)-9-[(1β,2α,3β)-2-Acetoxy-3-acetoxymethylcyclo butan-1-yl]-2-amino-6-chloropurine (45 mg, 0.13 mmol) was dissolved in ethanol (5 ml) and 10% palladium on carbon (75 mg) was added to the solution. The mixture was stirred at room temperature overnight under hydrogen atmosphere. After the catalyst was filtered off, the solvent was distilled off under reduced pressure. The residue was purified by preparative high performance liquid chromatography (ODS, water : acetonitrile = 80 : 20 (v/v)) to give (±)-9-[(1β,2α,3β)-2-acetoxy-3-acetoxymethylcyclobutan-1-yl]-2-aminopurine (12.7 mg, 31 %).

Step 2

Preparation of (±)-2-amino-9-[(1β,2α,3β)-2-hydroxy-3-hydroxymethylcyclobutan-1-yl]purine (Compound 4-1)

(±)-9-[(1β,2α,3β)-2-Acetoxy-3-acetoxymethylcyclobutan-1-yl]purine (12 mg, 38 µmol) was dissolved in methanol (1 ml) and potassium carbonate (12 mg, 87 µmol) was added to the solution followed by stirring

at 0 °C for 30 minutes. After neutralizing with 0.1N-hydrochloric acid, the reaction liquid was purified by Sephadex HP20 (water-50% methanol aqueous solution) to give (±)-2-amino-9-[(1$\beta$,2$\alpha$,3$\beta$)-2-hydroxy-3-hydroxymethylcyclobutan-1-yl]purine (8.6 mg, 97%).
NMR (200 MHz.FT, $CDCl_3$) $\delta$:
1.78 (1H, m), 2.23 (1H, m), 2.41 (1H,m), 3.60-3.80 (2H, m), 4.29 (1H, apparent t, J=7.7Hz), 4.49 (1H, apparent q, J=8.5Hz), 8.08 (1H, s), 8.42 (1H, s).
UV $\lambda_{max}(H_2O)$ nm:
pH 1, 249(sh), 313; pH 7, 242, 304; pH 13, 245(sh), 303.

Example 25

Preparation of (±)-9-[(1$\beta$,2$\alpha$,3$\beta$)-2-hydroxy-3-hydroxymethylcyclobutan-1-yl]hypoxanthine (Compound 4-2):

(±)-9-[(1$\beta$,2$\alpha$,3$\beta$)-2-Hydroxy-3-hydroxymethylcyclobutan-1-yl]adenine (9 mg, 38 $\mu$mol) and adenosine deaminase (30 mg, 27 units) were dissolved in 0.1 M phosphate buffer (pH 6.8) (9 ml). The solution was stirred at 30 °C for 5 days. The reaction liquid was purified by Sephadex HP20 (water-50% methanol aqueous solution) to give (±)-9-[(1$\beta$,2$\alpha$,3$\beta$)-2-hydroxy-3-hydroxymethylcyclobutan-1-yl]hypoxanthine (9 mg, 100%).
NMR (200 MHz.FT, $CD_3OD$) $\delta$:
2.01 (1H, m), 2.22 (1H, m), 2.45 (1H, m), 3.73-3.83 (2H, m), 4.47 (1H, apparent t, J=7.1Hz), 4.67 (1H, apparent q, J=8.4Hz), 8.05 (1H, s), 8.18 (1H, s).
UV $\lambda_{max}(H_2O)$ nm:
pH 1, 250; pH 7, 249; pH 13, 254.

Example 26

Preparation of 1-[(1$\beta$,3$\beta$)-3-hydroxymethylcyclobutan-1-yl]-5-methyl-2,4(1H,3H)-pyrimidinedione (Compound 3-21):

In an argon atmosphere, (±)-(1$\beta$,3$\beta$)-3-t-butyldiphenylsilyloxymethyl-1-methanesulfonyloxycyclobutane-(126 mg, 0.3 mmol) was dissolved in dimethylformamide (3 ml) and bistrimethylsilyloxy-5-methyluracil (325 mg, 1.2 mmol) was added to the solution. The mixture was stirred at 120 °C overnight. To the reaction mixture was added 0.2 M phosphate buffer (pH 7.0). The mixture was extracted with ethyl acetate. After the extract was dried over anhydrous sodium sulfate, the solvent was distilled off under reduced pressure. The residue was purified by silica gel column chromatography (methylene chloride : methanol = 50 : 1 (v/v)) to give crude 1-[(1$\beta$,3$\beta$)-3-t-butyldiphenylsilyloxymethylcyclobutan-1-yl]-5-methyl-2,4(1H,3H)-pyrimi dinedione (40 mg).

To a solution of this compound in methanol (1 ml) was added 4N-hydrochloric acid/dioxane (0.2 ml, 0.8 mmol). The mixture was stirred at room temperature overnight. After the solvent was distilled off under reduced pressure, ether soluble matters were removed. The remaining matter was separated and purified by preparative high performance liquid chromatography (ODS, 5% acetonitrile aqueous solution) and further Sephadex LH-20 column chromatography (methanol) to give 1-[(1$\beta$,3$\beta$)-3-hydroxymethylcyclobutan-1-yl]-5-methyl-2,4(1H,3H)pyrimidinedione (Compound 3-21).

Example 2-1

Preparation of 9-[(1$\beta$,3$\beta$)-3-hydroxymethylcyclobutan-1-yl]adenine (Compound 2-1):

Step 1

Preparation of 9-[(1β,3β)-3-t-butyldiphenylsilyloxymethylcyclobutan-1-yl]adenine

Adenine (81 mg, 0.6 mmol) was suspended in dimethylformamide (2 ml) in an argon atmosphere and 50% sodium hydride (28.8 mg, 0.6 mmol) was added to the suspension. The mixture was stirred at room temperature for an hour. To this solution was added a solution of (1α,3β)-3-t-butyldiphenylsilyloxymethyl-1-methanesulfonyloxycyclobutane (209 mg, 0.5 mmol) in dimethylformamide (3 ml). The mixture was stirred for 5 hours at 120 °C. To the reaction liquid was added 0.2 M phosphate buffer (pH 7.0) followed by extraction with ethyl acetate. After the extract was dried over anhydrous sodium sulfate, the solvent was distilled off under reduced pressure. The residue was purified by silica gel column chromatography (methylene chloride : methanol = 40 : 1 (v/v)) to give 9-[(1β,3β)-3-t-butyldiphenylsilyloxymethylcyclobutan-1-yl]adenine (102 mg, 45%).
NMR (400 MHz.FT, $CDCl_3$) δ:
1.10 (9H, s), 2.35-2.55 (3H, m), 2.55-2.66 (2H, m), 3.73 (2H, d, J=4.0Hz), 4.9 (1H, m), 5.70 (2H, brs, exchangeable with $D_2O$), 7.31-7.51 (6H, m), 7.64-7.73 (4H, m), 7.92 (1H, s), 8.34 (1H, s).

Step 2

Preparation of 9-[(1β,3β)-3-hydroxymethylcyclobutan-1-yl]adenine (Compound 2-1)

9-[(1β,3β)-3-t-Butyldiphenylsilyloxymethylcyclobutan-1-yl]adenine (94 mg, 0.21 mmol) was dissolved in methanol (2 ml) and 4N-hydrochloric acid/dioxane (0.3 ml, 1.2 mmol) was added to the solution. The mixture was stirred at room temperature overnight. After the solvent was distilled off under reduced pressure, water was added thereto and ether soluble matters were removed. After neutralizing with 0.1 N sodium hydroxide solution, the solvent was distilled off under reduced pressure. The crude product was separated and purified by Sephadex LH-20 column chromatography (methanol : water = 1 : 1 (v/v)) to give 9-[(1β,3β)-3-hydroxymethylcyclobutan-1-yl]adenine (Compound 2-1) (48 mg, quantitative).
NMR (400 MHZ.FT, $CD_3OD$) δ:
2.37-2.52 (3H, m), 2.57-2.73 (2H, m), 3.65 (2H, d, J=4.4Hz), 4.91 (1H, m), 8.23 (1H, s), 8.32 (1H, s).
UV $\lambda_{max}(H_2O)$ nm:
pH 1, 258; pH 7, 261; pH 13, 261.
HRMS (FAB) Calcd for $[C_{10}H_{13}N_5O+H]^+$; 220.1198. Found ; 220.1196.

Example 2-2

Preparation of 9-[(1β,3β)-3-hydroxymethylcyclobutan-1-yl]guanine (Compound 2-2):

Step 1

Preparation of 2-amino-9-[(1β,3β)-3-t-butyldiphenylsilyloxymethylcyclobutan-1-yl]-6-(2-methoxy)-ethoxypurine

2-Amino-6-(2-methoxy)ethoxypurine (168 mg, 0.8 mmol) was suspended in dimethylformamide (3 ml) in an argon atmosphere and lithium hydride (6.4 mg, 0.8 mmol) was added to the suspension. The mixture was stirred at room temperature for an hour. To this solution was added a solution of (1α,3β)-3-t-butyldiphenylsilyloxymethyl-1-methanesulfonyloxycyclobutane (280 mg, 0.67 mmol) in dimethylformamide (3 ml). The resulting mixture was stirred for 35 hours at 120 °C and at 140 °C for 4 hours. To the reaction liquid was added 0.2 M phosphate buffer (pH 7.0) followed by extraction with ethyl acetate. After the extract was dried over anhydrous sodium sulfate, the solvent was distilled off under reduced pressure. The residue was purified by silica gel column chromatography (methylene chloride : methanol = 20 : 1 (v/v)) to give 2-amino-9-[(1β,3β)-3-t-butyldiphenylsilyloxymethylcyclobutan-1-yl]-6-(2-methoxy)ethoxypurine (65 mg, 18%).
NMR (400 MHz.FT, $CDCl_3$) δ:
1.09 (9H, s), 2.30-2.48 (3H, m), 2.48-2.60 (2H, m), 3.43 (3H, s), 3.71 (2H, d, J=3.7Hz), 3.81 (2H, dd,

J = 4.8Hz, J = 5.1Hz), 4.64 (2H, dd, J = 4.8Hz, J = 5.1Hz), 4.68-4.84 (3H, m), 7.30-7.50 (6H, m), 7.58-7.70 (4H, m), 7.72 (1H, s).

Step 2

Preparation of 9-[(1β,3β)-3-hydroxymethylcyclobutan-1-yl]guanine (Compound 2-2)

To 2-amino-9-[(1β,3β)-3-t-butyldiphenylsilyloxymethylcyclobutan-1-yl]-6-(2-methoxy)ethoxypurine (63 mg, 0.12 mmol) was added 2N-hydrochloric acid. The mixture was heated to reflux for an hour. After the solvent was distilled off under reduced pressure, water was added thereto and ether soluble matters were removed. After neutralizing with 0.1 N sodium hydroxide solution, the solvent was distilled off under reduced pressure. The resude was repeatedly recrystallized from water to give 9-[(1β,3β)-3-hydroxymethylcyclobutan-1-yl]guanine (Compound 2-2) (7 mg, 25%).
NMR (400 MHz.FT, DMSO-$d_6$) δ:
2.14-2.30 (3H, m), 2.35-2.50 (2H, m), 3.46 (2H, t, J = 4.8Hz, changed to d(J = 4.8Hz) after addition of $D_2O$), 4.55 (2H, m, changed to 1H, m, after addition of $D_2O$), 6.41 (2H, brs, exchangeable with $D_2O$), 7.86 (1H, s), 10.56 (1H, brs, exchangeable with $D_2O$). UV $\lambda_{max}(H_2O)$ nm:
pH 1, 253, 277(sh); pH 7, 252, 272(sh); pH 13, 257(sh), 268.
HRMS (FAB) Calcd for $[C_{10}H_{13}N_5O_2 + H]^+$; 236.1147. Found ; 236.1130.

Reference Example

Preparation of of (1α,3β)-3-t-butyldiphenylsilyloxymethyl-1-methanesulfonyloxycyclobutane:

Step 1

Preparation of (1α,3β)-3-hydroxymethylcyclobutanol

(±)-(1α,2β,4α)-2-t-Butyldiphenylsilyloxymethyl-5-oxabicyclo[2.1.0]pentane (3.00 g, 7.17 mmol) was dissolved in tetrahydrofuran (10 ml) and 1M-tetrabutyl ammonium fluoride/tetrahydrofuran (8.6 ml, 8.6 mmol) was added to the solution. The mixture was stirred at room temperature for 3.5 hours. After the reaction liquid was concentrated, water was added to the concentrate to dissolve and the solution was washed with hexane. The aqueous phase was continuously extracted with ether for a day. After the ethereal extract was dried over anhydrous sodium sulfate, the solvent was distilled off under reduced pressure. The residue was dissolved in tetrahydrofuran (24 ml) and a solution of 70% sodium bis(2-methoxyethoxy)aluminum hydride (4.13 g, 14.3 mmol) in 8 ml of tetrahydrofuran was dropwise added to the resulting solution under ice cooling. Then, the mixture was stirred at room temperature for 7 hours. Isopropyl alcohol and water (2ml) was added to the reaction liquid. After stirring for a while, insoluble matters were filtered off. The solvent was removed from the filtrate by distillation under reduced ressure. The residue was separated and purified by silica gel column chromatography (methylene chloride : methanol = 20 : 1 (v/v)) to give (1α,3β)-3-hydroxymethylcyclobutanol (0.66 g, 90%).

Step 2

Preparation of (1α,3β)-3-trityloxymethylcyclobutanol

(1α,3β)-3-Hydroxymethylcyclobutanol (660 mg, 6.5 mmol) was dissolved in pyridine (5 ml) and trityl chloride (2.16 g, 7.75 mmol) was added to the solution. The mixture was stirred at room temperature overnight. The reaction liquid was concentrated and pyridine was removed therefrom. The residue was separated and purified by silica gel column chromatography (methylene chloride) to give crude (1α,3β)-3-trityloxymethylcyclobutanol (898 mg, 40%).

Step 3

Preparation of (1α,3β)-3-t-butyldiphenylsilyloxymethyl-1-methanesulfonyloxycyclobutanol

Crude (1α,3β)-3-trityloxymethylcyclobutanol (890 mg, 2.6 mmol) described above was dissolved in methylene chloride (10 ml). Under ice cooling, triethylamine (1.08 ml, 776 mmol) and methanesulfonyl chloride (445 mg, 3.88 mmol) were added to the solution. The reaction was stirred for 30 minutes. To the reaction liquid was added 0.2 M phosphate buffer (pH 7.0) followed by extraction with ether. After the ethereal extract was dried over anhydrous sodium sulfate, the solvent was distilled off under reduced pressure. The residue was dissolved in methanol (10 ml) and Amberlyst 15 (529 mg, 2.6 mmol) was added to the solution. The mixture was stirred at room temperature overnight. After insoluble matters were filtered off, the solvent was distilled off under reduced pressure. The residue was dissolved in dimethylformamide (8 ml). Under ice cooling, triethylamine (1.08 ml, 7.76 mmol) and t-butyldiphenylchlorosilane (1.07 g, 3.88 mmol) were added to the solution. The mixture was stirred overnight. Water was added to the reaction liquid and extraction was performed with ether. After drying over anhydrous sodium sulfate, the solvent was distilled off under reduced pressure. The residue was separated and purified by silica gel column chromatography (hexane : ether = 3 : 1 (v/v)) to give (1α,3β)-3-t-butyldiphenylsilyloxymethyl-1-methanesulfonyloxycyclobutanol (720 mg, 66%).
NMR (400 MHz.FT, $CDCl_3$) δ:
1.08 (9H, s), 2.34-2.48 (4H, m), 2.53 (1H, m), 2.96 (3H, s), 3.65 (2H, d, J=4.8Hz), 5.16 (1H, m), 7.37-7.49 (6H, m), 7.65 (4H, d, J=6.2Hz).

While the invention has been described in detail and with reference to specific embodiments thereof, it is apparent to one skilled in the art that various changes and modifications can be made therein without departing from the spirit and the scope of the present invention.

## Claims

1. A cyclobutane derivative represented by general formula (I):

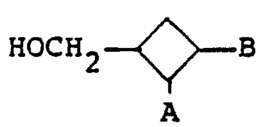

(I)

wherein A represents a hydrogen atom, a hydroxy group, an azido group or an amino group; and B represents a purine residue or a pyrimidine residue.

2. A cyclobutane derivative as claimed in claim 1, wherein said purine residue is:

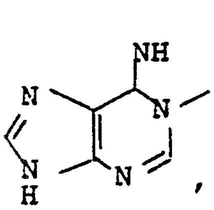 , 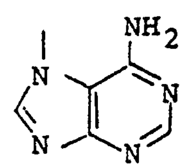 , 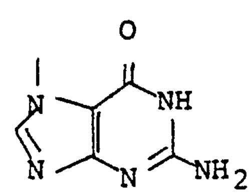 , 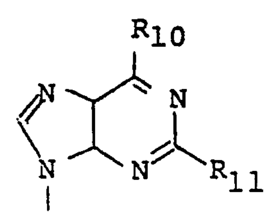

wherein $R_{10}$ represents a hydrogen atom, an amino group or a halogen atom and $R_{11}$ represents a hydrogen atom or an amino group, or,

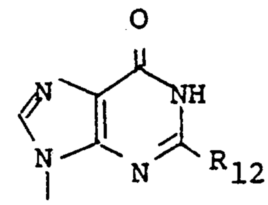

wherein $R_{12}$ represents a hydrogen atom or an amino group.

3. A cyclobutane derivative as claimed in claim 1, wherein said pyrimidine residue is:

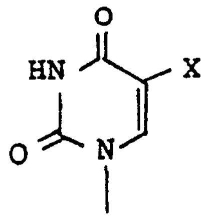

or

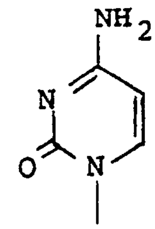

wherein X represents a hydrogen atom, a halogen atom or a lower alkyl group.

4. A cyclobutane derivative as claimed in claim 1, wherein A is a hydroxy group and B is a purine residue.

5. A cyclobutane derivative as claimed in claim 1, wherein A is a hydroxy group and B is:

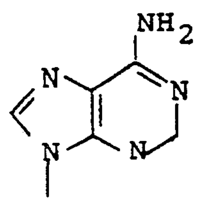

or

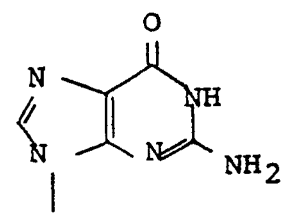

6. A cyclobutane derivative represented by general formula (II):

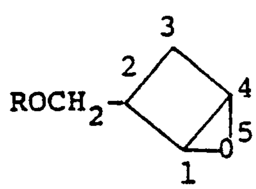

(II)

wherein R represents a hydrogen atom or a protective group.

7. A pharmaceutical composition containing cyclobutane derivatives of claim 1 as active ingredients, in association with a pharmaceutically acceptable, substantially non-toxic carrier or excipient.

8. A compound of claim 1 for use as a medicament.

9. A compound of claim 1 for use in treating or preventing diseases caused by virus or tumor.

10. Use of compounds of claim 1 for the manufacture of a medicament for therapeutic treatment of diseases caused by virus or tumor.